# EUROPEAN PATENT SPECIFICATION

(11) **EP 3 761 242 B1**
(45) Date of publication and mention of the grant of the patent: **24.06.2026**
(21) Application number: 20185727.3
(22) Date of filing: 06.12.2013
(51) Int. Cl.: G06Q 10/06, G06Q 50/02, G08B 17/00, G01N 33/24

(54) **FOREST SENSOR DEPLOYMENT AND MONITORING SYSTEM**
EINSATZ- UND -ÜBERWACHUNGSSYSTEM FÜR WALDSENSOREN
SYSTÈME DE DÉPLOIEMENT ET DE SURVEILLANCE DE CAPTEUR DE FORÊT

(30) Priority: 07.12.2012 US 201213708543
(43) Date of publication of application: 06.01.2021
(62) Divisional of application: 13196125.2
(73) Proprietor: The Boeing Company, Arlington, VA 22202 (US)
(72) Inventor: VIAN, John Lyle, Renton, Washington 98056 (US); SPINELLI, Charles B., Seattle, Washington 98124-2207 (US); TILLOTSON, Brian J., Chicago, Illinois 60606-2016 (US); ROE, George Michael, Seattle, Washington 98124-2207 (US); PRZYBYLKO, Joshua, Seattle, Washington 98124-2207 (US)
(74) Representative: Morrall, Jonathan Ian McLachlan

(56) References cited:
- EP-A1- 2 511 888
- WO-A1-96/29597
- JP-A- 2008 150 028
- US-A- 5 963 733
- US-A1- 2012 115 241

## Description

### BACKGROUND INFORMATION

### 1. Field:

The present disclosure relates generally to forest management and, in particular, to forest management operations. Still more particularly, the present disclosure relates to a method and apparatus for performing forest management operations.

### 2. Background:

Forestry management is a branch of forestry that includes many different aspects. These aspects may include environmental, economic, administrative, legal, and social aspects of managing a forest. Forestry management may consist of various techniques such as timber extraction, planting trees, replanting trees, cutting roads and pathways through forests, preventing fires in a forest, maintaining the health of the forest, and other suitable activities.

When performing these and other operations with respect to forest management, collecting information about the forest may be desired. For example, collecting information about the forest provides an ability to analyze the state of the forest as well as identify operations that may be performed.

The tools used to generate information for assessing the state of a forest may include, for example, without limitation, a clinometer, a data recorder, an increment borer, a wedge prism, a diameter tape, a global positioning system device, a tally meter, a laptop computer, and other suitable tools. These tools are used by forestry management personnel to perform various operations such as estimating numbers of trees present in an area, identifying the health of trees, identifying the age of trees, identifying tree spacing, identifying composition of soil samples, and other suitable operations.

With this information, an analysis of the information may be made to identify a state of the forest. This state of the forest may be a forest inventory. This forest inventory may provide results such as the value of the timber, expected cash flows from the timber, the amount of timber land existing, impacts of recreational use, risks of fire, improvements to increase the growth and value of the forest, the time period in which timber should be harvested, and other suitable results.

Currently, the process for collecting information for assessing the state of a forest is very time-consuming and complex. For example, collecting information may require tens of thousands or hundreds of thousands of sensor readings or observations made by forest management personnel for a particular location in the forest. With additional locations, even more information is collected. Moreover, collecting this information within desired time periods and as frequently as desired increases the time and effort needed.

Further, current processes also often rely on sampling when collecting information. Sampling may be performed in selected locations instead of from the entire forest. This type of information collection may be used when obtaining information over the whole forest and is more time-consuming than desired. Further, when sampling, errors may occur due to a lack of adequate information collection and analysis.

The collection of information is performed by forest management personnel using tools that often may require interpretation by the forest management personnel. As a result, different human operators may make different interpretations while making measurements. The lack of consistency of interpretations may lead to undesired results.

For example, two different people may decide that different types of samplings should be used based on two different measurements of tree spacing. As another example, when using a clinometer, measurement of the height of a tree using two different clinometers may produce different results. These differences may provide results that may not be as accurate as desired.

Further, the information may be inconsistent depending on the ability of forest management personnel to reach different portions of the forest. For example, access to certain locations within the forest may be infeasible for forestry management personnel. In these inaccessible regions, the information may be unavailable and as a result the state of the forest may not be identified as accurately as desired.

Additionally, the availability of forest management personnel to collect information may not be as great as desired in order to obtain a desired amount of information for performing an analysis. Additionally, this analysis may not be performed with a desired level of accuracy or with the use of as up-to-date information as desired.

As a result, collecting information needed to analyze the state of the forest is often much more complex and difficult than desired. With the number of pieces of information needed and the frequency at which the information is needed, the amount of forest management personnel needed to obtain this information may be infeasible due to the amount of personnel available or the costs associated with use of these personnel. Further, with the use of human operators to make measurements and observations, the information collected may not be as uniform or as accurate as desired.

Therefore, it would be desirable to have a method and apparatus that takes into account at least some of the issues discussed above, as well as other possible issues.

EP 2511888 A1, in accordance with its abstract, states a method and apparatus for managing fires. A control system is configured to receive fire related information from at least a first portion of a plurality of assets and analyze the fire-related information to generate a result. The control system is configured to coordinate an operation of a second portion of the plurality of assets using the result.

### SUMMARY

In one illustrative example, a forestry management system comprises a forestry manager. The forestry manager is configured to receive information relating to a number of soil conditions for a location in a forest from a sensor system (1024) deployed by a group of aerial vehicles and identify a mission based on the number of soil conditions.

In an illustrative embodiment, a forestry management system comprises sensor units and a group of aerial vehicles. The sensor units are configured to be deployed in a location, generate information about a number of soil conditions in the location, and transmit the information using wireless communications links. The group of aerial vehicles is configured to carry the sensor units and deploy the sensor units in the location. The sensor units include and deploy the sensor units in the location. The sensor units comprise soil sensor units, the soil sensor units being bottom heavy comprising pins and a housing, the pins and housing being weighted such that the pins extend into the ground when the soil sensor units are deployed through an airdrop.

In another illustrative embodiment, a method for managing a location is presented. Soil sensor units are deployed in the location in a forest from a group of aerial vehicles. Information is generated about a number of soil conditions in the location in the forest using the soil sensor units in the location. The information is transmitted from transmitters in the soil sensor units to a remote location for analysis. The sensor units comprise soil sensor units, the soil sensor units being bottom heavy comprising pins and a housing, the pins and housing being weighted such that the pins extend into the ground when the soil sensor units are deployed through an airdrop.

The features and functions can be achieved independently in various embodiments of the present disclosure or may be combined in yet other embodiments in which further details can be seen with reference to the following description and drawings.

### BRIEF DESCRIPTION OF THE DRAWINGS

The novel features believed characteristic of the illustrative embodiments are set forth in the appended claims. The illustrative embodiments, however, as well as a preferred mode of use, further objectives and features thereof, will best be understood by reference to the following detailed description of an illustrative embodiment of the present disclosure when read in conjunction with the accompanying drawings, wherein:
**Figure 1** is an illustration of a forestry management environment in accordance with an illustrative embodiment;
**Figure 2** is an illustration of a block diagram of a forestry management environment in accordance with an illustrative embodiment;
**Figure 3** is an illustration of data flow in a forestry manager in accordance with an illustrative embodiment;
**Figure 4** is an illustration of a block diagram of types of missions in accordance with an illustrative embodiment;
**Figure 5** is an illustration of a block diagram of a task in accordance with an illustrative embodiment;
**Figure 6** is an illustration of a block diagram of an autonomous vehicle in accordance with an illustrative embodiment;
**Figure 7** is an illustration of a block diagram of a positioning and map building sensor module in accordance with an illustrative embodiment;
**Figure 8** is an illustration of a block diagram of a sensor module in accordance with an illustrative embodiment;
**Figure 9** is an illustration of a block diagram of a support system in accordance with an illustrative embodiment;
**Figure 10** is an illustration of a block diagram of a forestry management environment in accordance with an illustrative embodiment;
**Figure 11** is an illustration of a block diagram of ground-based sensor unit in accordance with an illustrative embodiment;
**Figure 12** is an illustration of a deployment of a sensor system for obtaining soil information in accordance with an illustrative embodiment;
**Figure 13** is an illustration of a soil sensor unit in accordance with an illustrative embodiment;
**Figure 14** is an illustration of a soil sensor unit in accordance with an illustrative embodiment;
**Figure 15** is an illustration of a forest area in accordance with an illustrative embodiment;
**Figure 16** is an illustration of a soil sensor aerial deployment unit in accordance with an illustrative embodiment;
**Figure 17** is an illustration of a model of decision making for planting trees in accordance with an illustrative embodiment;
**Figure 18** is an illustration of a model of decision making for in-filling recently planted areas of a forest in accordance with an illustrative embodiment;
**Figure 19** is an illustration of a flowchart of a process for managing a forest in accordance with an illustrative embodiment;
**Figure 20** is an illustration of a flowchart of a process for processing information received from assets in accordance with an illustrative embodiment;
**Figure 21** is an illustration of a flowchart of a process for coordinating the operation of assets in accordance with an illustrative embodiment;
**Figure 22** is an illustration of a flowchart of a process for managing a location in accordance with an illustrative embodiment;
**Figure 23** is an illustration of a flowchart of a process for obtaining information about a number of soil conditions in a location in a forest in accordance with an illustrative embodiment;
**Figure 24** is an illustration of a flowchart of a process for generating a mission in accordance with an illustrative embodiment;
**Figure 25** is an illustration of a flowchart of a decision making process for generating and performing a mission in accordance with an illustrative embodiment;
**Figure 26** is an illustration of a flowchart of a decision making process for generating and performing forest operations in a mission in accordance with an illustrative embodiment; and
**Figure 27** is an illustration of a block diagram of a data processing system in accordance with an illustrative embodiment.

### DETAILED DESCRIPTION

The illustrative embodiments recognize and take into account one or more different considerations. For example, the illustrative embodiments recognize and take into account that the currently used systems for collecting information about a forest may not provide as much information or as accurate information as desired for performing forestry management.

Thus, the illustrative embodiments provide a method and apparatus for managing a forest. In one illustrative embodiment, a forestry manager is configured to receive information about a forest from a group of autonomous vehicles. The forestry manager analyzes the information to generate a result about a state of the forest. The forestry manager also coordinates the operation of the group of autonomous vehicles using the result.

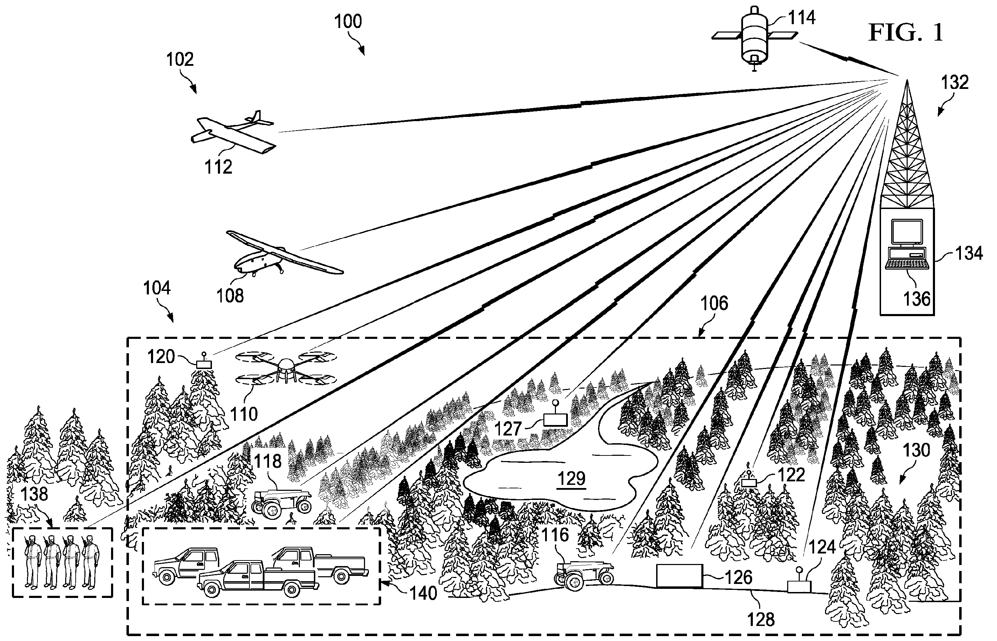

With reference now to the figures and, in particular, with reference to **Figure 1****,** an illustration of a forestry management environment is depicted in accordance with an illustrative embodiment. As depicted, forestry management environment **100** includes assets **102.**

Assets **102** generate information about locations in forest **104** such as location **106.** In this illustrative example, assets **102** include unmanned vehicles such as unmanned aerial vehicle **108,** unmanned aerial vehicle **110,** unmanned aerial vehicle **112,** satellite **114,** unmanned ground vehicle **116,** and unmanned ground vehicle **118.** Additionally, assets **102** also may include sensor systems such as ground-based sensor unit **120,** ground-based sensor unit **122,** ground-based sensor unit **124,** and ground-based sensor unit **127.** Support system **126** also may be present to provide support for the unmanned vehicles.

As depicted, unmanned aerial vehicle **108** and unmanned aerial vehicle **110** may operate at lower altitudes as compared to unmanned aerial vehicle **112.** For example, unmanned aerial vehicle **108** and unmanned aerial vehicle **110** may operate from ground **128** of forest **104** up to altitudes of about 2,000 feet in these illustrative examples. Unmanned aerial vehicle **112** may operate at higher altitudes such as altitudes over 30,000 feet depending on the particular implementation.

As depicted, unmanned aerial vehicle **108,** unmanned aerial vehicle **110,** and unmanned aerial vehicle **112** use onboard sensors to generate information about location **106** in forest **104.** Satellite **114** also may use onboard sensors to generate information about location **106** in forest **104.**

In these illustrative examples, unmanned ground vehicle **116** and unmanned ground vehicle **118** may move on ground **128** of forest **104.** Unmanned ground vehicle **116** and unmanned ground vehicle **118** also may generate information about location **106** in forest **104** using onboard sensors.

Additionally, ground-based sensor unit **120,** ground-based sensor unit **122,** ground-based sensor unit **124,** and ground-based sensor unit **127** are present in location **106** in forest **104** and also generate information about location **106** in forest **104.** In these illustrative examples, ground-based sensor unit **120** and ground-based sensor unit **122** may be placed in trees **130.** Ground-based sensor unit **124** may be located on ground **128** in forest **104.**

In some illustrative examples, ground-based sensors may be operated near water. In these illustrative examples, ground-based sensor unit **127** may be placed near body of water **129.** In these illustrative examples, ground-based sensor unit **127** may be used to measure the water quality of body of water **129.**

In these illustrative examples, support system **126** may be a stationary structure or a mobile structure. For example, support system **126** may be a base, a station, a van, or other structure that provides support for at least one of unmanned aerial vehicle **108,** unmanned aerial vehicle **110,** unmanned ground vehicle **116,** and unmanned ground vehicle **118** to recharge batteries, exchange batteries, or otherwise obtain power to operate.

As used herein, the phrase "at least one of", when used with a list of items, means different combinations of one or more of the listed items may be used and only one of each item in the list may be needed. For example, "at least one of item A, item B, and item C" may include, without limitation, item A or item A and item B. This example also may include item A, item B, and item C or item B and item C.

Additionally, support system **126** also may provide shelter from the environment, repair facilities, and provide other services to one or more of unmanned aerial vehicles or unmanned ground vehicles. In this illustrative example, support system **126** may operate in an automated fashion without need for human intervention. In some cases, support system **126** also may store information that may be generated by unmanned aerial vehicle **108,** unmanned aerial vehicle **110,** unmanned ground vehicle **116,** or unmanned ground vehicle **118.**

The information generated by assets **102** may be sent over wireless communications links **132** to control station **134.** Forestry manager **136** in control station **134** is configured to process the information generated by assets **102.**

Additionally, forestry manager **136** also may coordinate the operation of assets **102** in these illustrative examples. This coordination may include directing the movement of assets **102,** identifying locations in forest **104** for monitoring, and other suitable operations that may be performed by assets **102.** In some illustrative examples, forestry manager **136** and the components in forestry manager **136** may be distributed between control station **134** and other components in forestry management environment **100.**

For example, forestry manager **136** may be distributed between control station **134** and support system **126.** For example, a portion of forestry manager **136** may be located in support system **126** while another portion of forestry manager **136** may be located in control station **134.** In this case, the components in forestry manager **136** may be in communication with each other over wireless communications links **132.**

In other illustrative examples, forestry manager **136** may be distributed within computers in assets **102.** For example, forestry manager **136** may be distributed in control station **134,** unmanned aerial vehicle **112,** and unmanned ground vehicle **116.**

In some illustrative examples, assets **102** also may include personnel **138** and manned vehicles **140.** Personnel **138** and manned vehicles **140** may supplement operations performed by the unmanned assets in these illustrative examples. Additionally, forestry manager **136** also may provide directions to at least one of personnel **138** and manned vehicles **140** to coordinate the operation of these assets. In this manner, the operation of different assets, both unmanned assets and manned assets, are coordinated by forestry manager **136** in control station **134.**

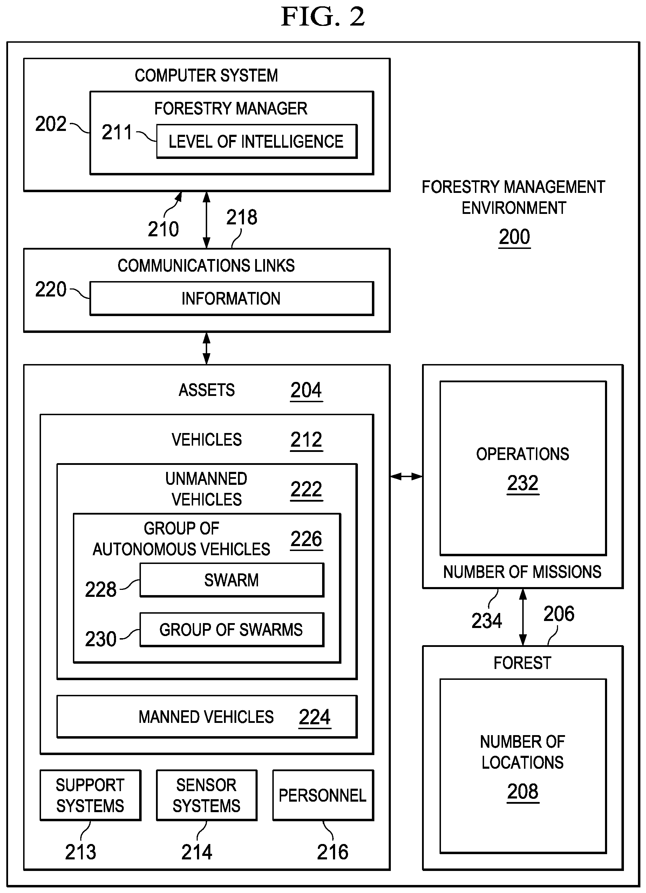

With reference now to **Figure 2****,** an illustration of a block diagram of a forestry management environment is depicted in accordance with an illustrative embodiment. Forestry management environment **100** in **Figure 1** is an example of one implementation for forestry management environment **200** in **Figure 2****.**

In this illustrative example, forestry management environment **200** includes forestry manager **202** and assets **204.** Forestry manager **202** and assets **204** are configured to manage forest **206.**

In particular, forestry manager **202** may be configured to manage number of locations **208** in forest **206.** As used herein, a "number of" when used with reference to items means one or more items. For example, number of locations **208** is one or more locations. Number of locations **208** may be a portion of forest **206** or may include all of forest **206.**

In this illustrative example, forestry manager **202** may be implemented using hardware, software, or a combination of the two. When software is used, the operations performed by forestry manager **202** may be implemented in program code configured to run on a processor unit. When hardware is employed, the hardware may include circuits that operate to perform the operations in forestry manager **202.**

For example, the hardware may take the form of a circuit system, an integrated circuit, an application specific integrated circuit (ASIC), a programmable logic device, or some other suitable type of hardware configured to perform a number of operations. With a programmable logic device, the device is configured to perform the number of operations. The device may be reconfigured at a later time or may be permanently configured to perform the number of operations. Examples of programmable logic devices include, for example, a programmable logic array, a programmable array logic, a field programmable logic array, a field programmable gate array, and other suitable hardware devices. Additionally, the processes may be implemented in organic components integrated with inorganic components and/or may be comprised entirely of organic components excluding a human being. For example, the processes may be implemented as circuits in organic semiconductors.

As depicted, forestry manager **202** may be implemented within computer system **210.** Computer system **210** is one or more computers. When more than one computer is present in computer system **210,** those computers may be in communication with each other over a communications medium such as a network.

These computers may be in the same geographic location or separate geographic locations depending on the particular implementation. Further, in some illustrative examples, a portion or all of computer system **210** may be mobile. For example, one or more computers in computer system **210** may be located in or carried by a platform such as a truck, an aircraft, a ship, a human operator, or some other suitable platform.

In these illustrative examples, forestry manager **202** may have level of intelligence **211.** Level of intelligence **211** may vary depending on the implementation of forestry manager **202.** In some cases, forestry manager **202** may be a computer program that receives input from a human operator and provides output to a human operator.

In other illustrative examples, level of intelligence **211** may be higher such that input from a human operator may be unnecessary. For example, an artificial intelligence system and other suitable types of processors may provide a desired level of intelligence for level of intelligence **211** in forestry manager **202.** In particular, the artificial intelligence system may include an expert system, a neural network, simple heuristics, fuzzy logic, Bayesian networks, or some other suitable type of system that provides a desired level of intelligence for level of intelligence **211** in forestry manager **202.**

As depicted, assets **204** include at least one of vehicles **212,** support systems **213,** sensor systems **214,** and personnel **216.** In these illustrative examples, assets **204** may communicate with forestry manager **202** and with each other using communications links **218.**

For example, assets **204** may generate information **220.** Information **220** may be sent to forestry manager **202** over communications links **218.** Additionally, information **220** may be exchanged between assets **204** over communications links **218.** In these illustrative examples, information **220** may include, for example, information about at least one of vegetation, soil conditions, wildlife, air quality, pollution, temperature, rainfall, and other suitable types of information.

As depicted, vehicles **212** may include unmanned vehicles **222** and manned vehicles **224.** Vehicles **212** may generate information **220** as vehicles **212** travel through or near number of locations **208** in forest **206.** Unmanned vehicles **222** may be remotely controlled by personnel **216** or may be autonomous. Unmanned vehicles **222** may be selected from at least one of an unmanned aerial vehicle, an unmanned ground vehicle, an unmanned water vehicle, and other suitable types of unmanned vehicles. When unmanned vehicles **222** are unmanned water vehicles, the unmanned water vehicles may be used in a lake, a pond, a river, or some other suitable type of body of water near the forest. Manned vehicles **224** are vehicles that may carry personnel **216** and are operated by personnel **216.**

Additionally, unmanned vehicles **222** may include group of autonomous vehicles **226.** An autonomous vehicle is a vehicle that operates without intervention from a human operator. In these illustrative examples, an autonomous vehicle may be remotely controlled or may have a desired level of intelligence. As used herein, a "group" when used with reference to items means one or more items. For example, group of autonomous vehicles **226** is one or more autonomous vehicles. Group of autonomous vehicles **226** may be configured to operate as swarm **228** or group of swarms **230** in these illustrative examples.

Support systems **213** are hardware systems configured to provide support for vehicles **212.** In particular, support systems **213** may provide support for unmanned vehicles **222.** For example, support systems **213** may provide shelter, power, maintenance, and other types of support for unmanned vehicles **222.**

Sensor systems **214** are also configured to generate information **220.** In these illustrative examples, sensor systems **214** are in fixed locations in number of locations **208** or near number of locations **208** in forest **206.**

Personnel **216** may perform operations including generation of information **220.** For example, personnel **216** may carry sensors, operate manned vehicles **224,** or operate unmanned vehicles **222** that are not within group of autonomous vehicles **226.**

In this illustrative example, forestry manager **202** is configured to coordinate operations **232** performed by assets **204.** Coordinating the operation of group of autonomous vehicles **226** to perform collection of information **220** may include collecting information **220** in at least one of a selected area in the forest, over a selected period of time, and with a selected level of detail.

Coordinating operations **232** also involves directing assets **204** to perform number of missions **234.** Coordinating assets **204** to perform number of missions **234** may reduce redundancy or overlap in the operation of assets **204** when redundancy or overlap is undesirable.

Further, coordinating assets **204** to perform number of missions **234** may include directing assets **204** by, for example, without limitation, sending at least one of a command, a message, a mission, a task, data, and other information that directs and/or gives guidance in performing number of missions **234.** This coordination may occur in a manner such that operations **232** are performed such that some or all of assets **204** may work together, as a single group, or in multiple groups to perform number of mission **234.**

For example, forestry manager **202** may coordinate swarm **228** by sending commands to each of the autonomous vehicles in swarm **228.** In these illustrative examples, swarm **228** is a plurality of autonomous vehicles, such as group of autonomous vehicles **226,** which coordinate the performance of operations **232** with each other.

In yet other illustrative examples, forestry manager **202** may send tasks to each of the autonomous vehicles in swarm **228.** Thus, group of autonomous vehicles **226** may use tasks and perform operations based on the tasks sent to each of the vehicles in group of autonomous vehicles **226.**

In still another illustrative example, forestry manager **202** may send tasks to manned vehicles **224** in addition to swarm **228** of group of autonomous vehicles **226.** When commands are sent to manned vehicles **224,** these commands may be viewed by personnel **216** in manned vehicles **224** in these illustrative examples. Further, personnel **216** in manned vehicles **224** may use these commands as input to control manned vehicles **224.** In other illustrative examples, personnel **216** may use these commands to perform operations on foot.

As depicted, forestry manager **202** may direct swarm **228** to a particular location in number of locations **208** and direct swarm **228** to generate information **220** in the particular location. In another example, forestry manager **202** may direct swarm **228** to travel along a selected path.

In a similar fashion, forestry manager **202** may send information for different missions in number of missions **234** to group of swarms **230.** Thus, a swarm in group of swarms **230** may perform the same or different missions from other swarms in group of swarms **230.**

With the use of forestry manager **202** and unmanned vehicles **222,** the amount of personnel **216** may be reduced as compared to currently used systems. Further, when personnel **216** are limited, the use of unmanned vehicles **222** and, in particular, group of autonomous vehicles **226,** may increase the ability to collect a desired amount of information **220** along with a desired accuracy and consistency for information **220** as compared to currently used systems for collecting information from number of locations **208** in forest **206.**

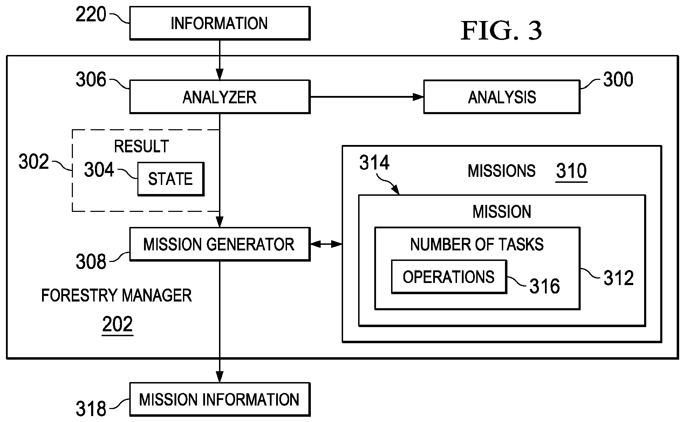

Turning now to **Figure 3****,** an illustration of data flow in a forestry manager is depicted in accordance with an illustrative embodiment. In this depicted example, forestry manager **202** analyzes information **220** received from assets **204 in** **Figure 2****.** In particular, forestry manager **202** performs analysis **300** using information **220.**

In these illustrative examples, analyzer **306** performs analysis **300** to generate result **302.** Result **302** includes state **304** for forest **206** in **Figure 2****.** State **304** may be, for example, without limitation, the state of forest health, forest inventory, safety risks, illegal activity, and other types of states of forest **206.**

In these illustrative examples, analysis **300** of information **220** may be performed in a number of different ways to obtain result **302.** Analysis **300** may include inspecting, cleaning, transforming, modeling, and other operations with respect to information **220.**

As depicted, analysis **300** may be performed using any currently available analysis technique for data. For example, without limitation, analyzer **306** may perform analysis **300** of information **220** using image processing systems, light detection and ranging systems, geographic information systems, visual inspection systems, or other suitable types of systems. In particular, analyzer **306** may perform analysis **300** to obtain result **302** by using data clustering and correlation, anomaly detection, statistical and prognostic methods, and other suitable types of data analysis techniques. In some cases, analysis **300** also may include simulations using models of forest **206.**

In other illustrative examples, result **302** may be obtained using a cloud detection system with trajectory generation methods and airborne laser scanners to provide timely and complete coverage of forest **206.** Specifically, forest manager **202** may perform analysis **300** on information **220** using this cloud detection system to obtain result **302** over a larger area of forest **206** than may be feasible with the use of currently available systems.

With result **302,** mission generator **308** identifies missions **310.** Additionally, mission generator **308** also may identify missions **310** without result **302.** For example, prior to obtaining information **220** about forest **206,** mission generator **308** may generate one or more of missions **310** to obtain information **220** for analysis **300** by analyzer **306.** In this illustrative example, a mission is a goal or objective. In other words, a mission in missions **310** may be one or more goals or objectives.

For example, mission generator **308** identifies number of tasks **312** for mission **314** in missions **310.** A task is a piece of work that is performed to achieve a mission. A task may be comprised of operations **316** that are performed for the piece of work.

Number of tasks **312** is one or more tasks to be performed by assets **204** in **Figure 2****.** Each task in number of tasks **312** may include one or more operations in operations **316.** Mission generator **308** also may identify operations **316** for number of tasks **312** in generating mission **314.**

For example, a mission may be to gather more information **220** about forest **206.** The task in number of tasks **312** may be to monitor a particular location in number of locations **208** in forest **206.** Operations **316** for the tasks may be to fly a selected path over the location in number of locations **208** in forest **206** and generate images of the location.

In these illustrative examples, mission generator **308** assigns at least one of mission **314,** number of tasks **312,** and operations **316** to assets **204.** In other words, mission generator **308** may send different levels of mission information **318** to assets **204** depending on the intelligence of assets **204** that are to perform mission **314.** This mission information 318 may be the same mission information **318** sent to each of assets **204.** In other illustrative examples, mission information **318** may be different for each of the assets in assets **204.** In this manner, forestry manager may coordinate the performance of missions **310** by sending mission information **318.**

For example, mission generator **308** may generate mission **314** with number of tasks **312.** Mission generator **308** assigns number of tasks **312** to group of autonomous vehicles **226** in **Figure 2****.** With the assignment of number of tasks **312,** mission generator **308** sends mission information **318** to group of autonomous vehicles **226** to perform number of tasks **312** in mission **314.**

In this manner, group of autonomous vehicles **226** may perform number of tasks **312** to complete all or a portion of mission **314.** In some illustrative examples, mission generator **308** may assign a portion of number of tasks **312** to group of autonomous vehicles **226** and another portion of number of tasks **312** to manned vehicles **224** in **Figure 2****.** In this case, both group of autonomous vehicles **226** in unmanned vehicles **222** and manned vehicles **224** use mission information **318** to complete a portion of mission **314.**

For example, when coordinating trespasser response, mission **314** may be to assist law enforcement. Mission generator **308** may send mission information **318** to unmanned aerial vehicle **108** to track an intruder, unmanned aerial vehicle **110** to take video footage of a crime scene, and manned vehicles **140** to bring personnel **138** in **Figure 1** to the location of the trespassing event. In this manner, each of assets **102** performs a portion of number of tasks **312** to complete mission **314** using mission information **318** sent by mission generator **308.**

Mission information **318** may take various forms. For example, mission information **318** may include commands, tasks, data, and other suitable information. As an example, number of tasks **312** may be sent in mission information **318** to group of autonomous vehicles **226** such that group of autonomous vehicles **226** performs operations **316** necessary to achieve number of tasks **312** in mission **314.** In other cases, mission information **318** may include commands needed to perform operations **316** to complete number of tasks **312** for missions **310.**

In some cases, an identification of mission **314** in mission information **318** may be sufficient for assets **204** to perform mission **314.** In other cases, number of tasks **312** may be assigned to assets **204.**

For example, the assignment may involve assigning number of tasks **312** to one or more of group of autonomous vehicles **226.** In other cases, number of tasks **312** may be assigned by sending number of tasks **312** to group of autonomous vehicles **226.** Group of autonomous vehicles **226** may coordinate and make their own assignments after receiving number of tasks **312.**

In other words, the assignment of number of tasks **312** may be to group of autonomous vehicles **226** as a whole, or to individual autonomous vehicles in group of autonomous vehicles **226.** When the assignment of number of tasks **312** is to group of autonomous vehicles **226** as a whole, specific tasks in number of tasks **312** may be distributed to autonomous vehicles in group of autonomous vehicles **226** based on the location of the autonomous vehicles, the capability of the autonomous vehicles, the response time of the autonomous vehicles, or some other suitable parameters.

In another illustrative example, mission generator **308** may send an identification of operations **316** to be performed by different assets in assets **204.** These different assets may be, for example, unmanned vehicles **222** and sensor systems **214.** These operations **316** may be at various levels and may be as detailed as particular commands on direction of movement, when to collect information, and other operations.

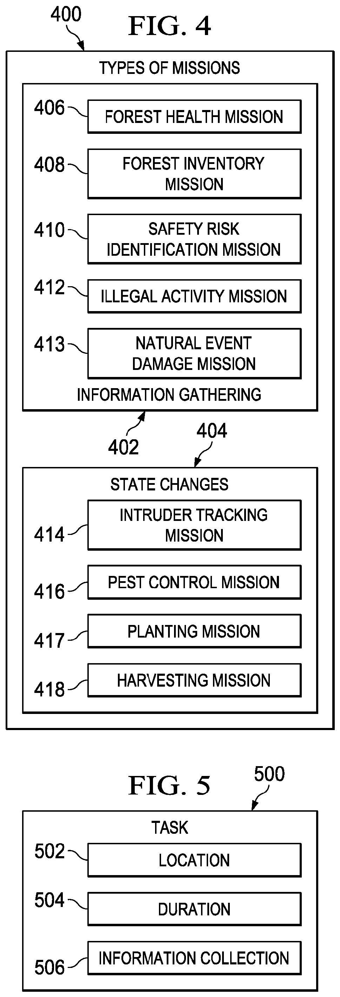

Turning now to **Figure 4****,** an illustration of a block diagram of types of missions is depicted in accordance with an illustrative embodiment. In this depicted example, types of missions **400** are examples of missions **310** in **Figure 3****.**

Types of missions **400** may comprise at least one of information gathering **402** and state changes **404.** Information gathering **402** comprises missions for obtaining information **220** in **Figure 2****.** State changes **404** comprise missions for causing a change in state **304** in **Figure 3** identified for forest **206** by forestry manager **202** in **Figure 2****.** In these illustrative examples, information gathering **402** may include at least one of forest health mission **406,** forest inventory mission **408,** safety risk identification mission **410,** illegal activity mission **412,** and natural event damage mission **413.**

In this illustrative example, forest health mission **406** is configured to generate information **220** that may be used to identify the health of a location within forest **206.** Forest health mission **406** may, for example, obtain information about trees in a location in forest **206.** In particular, forest health mission **406** may identify a biodiversity of trees and other vegetation in forest **206.**

Additionally, forest health mission **406** may be used to generate information **220** about spacing between trees. This forest health mission **406** may identify a presence of foreign species with respect to trees. In other words, types of species of trees that are not normally present in forest **206** may be identified using forest health mission **406.** Additionally, pests, infection, and other information about trees in forest **206** may be identified through information **220** generated from forest health mission **406.**

Forest health mission **406** may also collect information **220** that identifies the impact of human activity in forest **206.** For example, forest health mission **406** may identify information about unmanaged recreation, hunting, and local agriculture activities in forest **206.**

Further, forest health mission **406** also may generate information **220** used to identify the impact of natural events on forest **206.** These natural events may include storms, fires, and other events that may occur naturally in forest **206.**

Additionally, forest health mission **406** may generate information **220** about the health of vegetation on the floor of forest **206.** With this type of mission, information about wildlife within forest **206** and the health of wildlife within forest **206** may be generated.

In these illustrative examples, forest inventory mission **408** may be used to generate information **220** used to classify land within forest **206.** For example, forest inventory mission **408** may generate information used to identify a volume of wood that may be harvestable from forest **206.** Additionally, carbon sequestration may be identified during forest inventory mission **408.** In other words, the capture of carbon dioxide in forest **206** by trees and vegetation may be identified through forest inventory mission **408.**

With safety risk identification mission **410,** information **220** about safety risks such as a presence of fire may be included in this type of mission. In these illustrative examples, a "safety risk" is a risk of harm to forest **206** as a whole, wildlife or vegetation within forest **206,** humans, or a combination thereof. Thus, safety risk identification mission **410** is used to generate information **220** about the safety risks within forest **206.**

In some illustrative examples, safety risk identification mission **410** may generate information used to identify hazards to the public. This information may be used to identify what areas may be accessible by the public in forest **206.** In this manner, safety risks may be decreased within forest **206.** For example, when an area is determined to be a safety risk to the public by safety risk identification mission **410,** forest manager **202** in **Figure 2** may send one of assets **204** to block off that area to the public.

Illegal activity mission **412** is used to generate information **220** that may be used to identify various illegal activities within forest **206.** These illegal activities may include, for example, without limitation, poaching of timber, poaching of wildlife, illegal drug operations, trespassing in secured areas, squatting, and other illegal activities.

As depicted, natural event damage mission **413** may be used to generate information **220** about the damage that may be present after a natural event. For example, when a flood occurs in forest **206,** information **220** about damage caused by the flood may be needed. In this case, forest manager **202** may send one of assets **204** to gather information **220** about state changes **404** resulting from the flood. Of course, forest manager **202** may send one of assets **204** to gather information **220** about other types of natural events such as, for example, without limitation, fire, wind, ice, snow, earthquake, tornado, or some other type of natural event.

In these illustrative examples, state changes **404** include missions that are used to change state **304** of forest **206.** The change in state **304** may be for a portion or all of forest **206.** As depicted, state changes **404** may include various types of missions **400.** For example, state changes **404** may include at least one of intruder tracking mission **414,** pest control mission **416,** planting mission **417,** harvesting mission **418,** and other suitable types of missions **400.**

In these illustrative examples, intruder tracking mission **414** is a mission in which assets **204** are coordinated to identify and track an intruder within forest **206.** Pest control mission **416** may be used to control pests that may affect the health of forest **206** in an undesired manner. Pest control mission **416** may be used to send assets **204** to forest **206** to perform operations **316** to control or eliminate pests that may be in forest **206.**

For example, assets **204** may distribute chemicals, electrical agents, and other components to control pests that may be present in forest **206.** These pests may be vegetation, wildlife, or other types of pests.

In this illustrative example, planting mission **417** may be performed to plant trees in forest **206.** In these illustrative examples, planting mission **417** may include planting seedlings of trees in number of locations **208** of forest **206.** Number of locations **208** may be one or more locations in which open areas are present in forest **206** or in which trees are present, but the density of the trees is not as great as desired.

Harvesting mission **418** may be performed to harvest trees in forest **206.** Assets **204** may be assets configured to harvest trees that have been identified in particular locations in forest **206.** For example, tree harvesters in vehicles **212** in **Figure 2** may be used to harvest trees in forest **206.** These tree harvesters may take the form of autonomous vehicles within group of autonomous vehicles **226.**

The illustration of types of missions **400** in **Figure 4** is only presented as an example of some types of missions that may be present in missions **310.** The examples of types of missions **400** are not meant to imply limitations to other types of missions that may be used. Further, in some cases, only some of the missions illustrated in types of missions **400** may be used rather than all of the types of missions in types of missions **400.** The tasks and the operations performed for each of types of missions **400** may vary and may be implemented in numerous different ways depending on the makeup of forest **206** and the particular situation.

With reference now to **Figure 5****,** an illustration of a block diagram of a task is depicted in accordance with an illustrative embodiment. In this depicted example, task **500** is an example of a task that may be used to implement one or more of number of tasks **312** in **Figure 3****.**

As depicted, task **500** may have a number of different components. In this illustrative example, task **500** includes location **502,** duration **504,** and information collection **506.**

Location **502** is a location in which task **500** is to be performed. Location **502** may be defined as a geographic area, a physical volume, or a path. For example, location **502** may define an area on the ground in which the task is to be performed. In other illustrative examples, location **502** also may define a height in which information **220** in **Figure 2** is to be collected. In other illustrative examples, location **502** may be defined as a path that is to be travelled by the asset for the task.

Duration **504** identifies a period of time during which the task is to be performed. Duration **504** may include a start time and an end time.

In some illustrative examples, duration **504** may be defined based on an amount of power remaining in the asset for performing the task. In some cases, duration **504** may be defined as an amount of information **220** collected, a type of information **220** collected, or based on some other parameter other than time. Of course, a combination of these different types of measurements for duration **504** also may be used.

Information collection **506** identifies the type of information **220** to be collected and may also identify the manner in which information **220** is to be collected. In this case, information **220** may include information such as images, temperature readings, humidity readings, sample collections, and other suitable types of information. Further, information collection **506** also may define a frequency at which information **220** is to be collected.

Further, information collection **506** also may define the granularity of information **220** to be collected. For example, information collection **506** may define a higher granularity such that information **220** generates images of the height, straightness, taper, and volume of trees. In other illustrative examples, a lower granularity may merely comprise generating images of the location rather than more detailed measurements of trees in the location. Of course, any granularity may be defined in information collection **506** for task **500.**

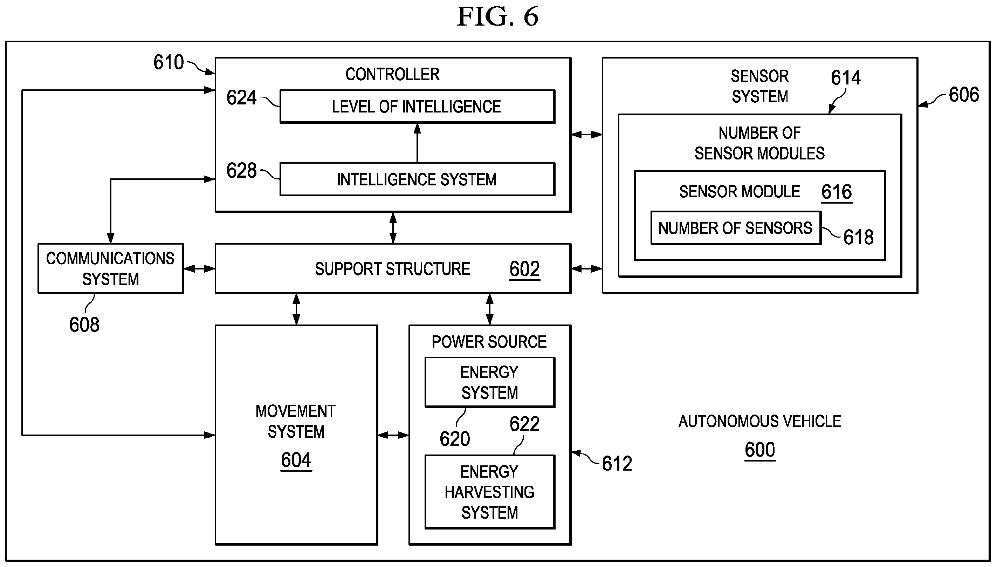

Turning now to **Figure 6****,** an illustration of a block diagram of an autonomous vehicle is depicted in accordance with an illustrative embodiment. In this depicted example, autonomous vehicle **600** is an example of one implementation for an autonomous vehicle within group of autonomous vehicles **226** in **Figure 2****.** Unmanned aerial vehicle **108,** unmanned aerial vehicle **110,** unmanned aerial vehicle **112,** unmanned ground vehicle **116,** and unmanned ground vehicle **118** are physical examples of unmanned vehicles that may be implemented as autonomous vehicles using components in autonomous vehicle **600.**

In this illustrative example, autonomous vehicle **600** includes a number of different components. For example, autonomous vehicle **600** includes support structure **602,** movement system **604,** sensor system **606,** communications system **608,** controller **610,** and power source **612.**

Support structure **602** provides a structure for physical support of the other components in autonomous vehicle **600.** Support structure **602** may be, for example, at least one of a frame, a housing, a body, and other suitable types of structures.

Movement system **604** is associated with support structure **602** and is configured to provide movement for autonomous vehicle **600.** Movement system **604** may take various forms. For example, movement system **604** may include at least one of legs, wheels, tracks, and other suitable types of mechanisms for moving autonomous vehicle **600.**

Sensor system **606** is a system associated with support structure **602.** Sensor system **606** is configured to generate information about the environment around autonomous vehicle **600.** Sensor system **606** may include many types of sensors.

In these illustrative examples, sensor system **606** may include number of sensor modules **614.** In these illustrative examples, a sensor module in number of sensor modules **614** is removable. In other words, one sensor module may be swapped out for another sensor module in number of sensor modules **614** in sensor system **606** in autonomous vehicle **600.**

In this manner, creator versatility may be provided for autonomous vehicle **600.** In particular, a sensor module in number of sensor modules **614** may be selected for use by autonomous vehicle **600** depending on the mission or task assigned to autonomous vehicle **600.** Further, with the use of number of sensor modules **614,** the weight of autonomous vehicle **600** may be reduced by reducing the number of sensors in sensor system **606** only to those needed for a particular mission or task.

For example, sensor module **616** may be comprised of number of sensors **618.** The composition of number of sensors **618** may be selected for the particular type of mission or task to be performed.

Communications system **608** is associated with support structure **602.** As depicted, communications system **608** is configured to provide communications between autonomous vehicle **600** and another device. This other device may be, for example, one of other assets in assets **204,** computer system **210,** forestry manager **202,** and other suitable components. The communications may be wireless communications in these illustrative examples. In some cases, a wired communications interface may also be present.

Power source **612** is associated with support structure **602.** Power source **612** is configured to provide power for the other components in autonomous vehicle **600.** Power source **612** may take a number of different forms. For example, power source **612** may include at least one of energy system **620** and energy harvesting system **622.**

In this illustrative example, energy system **620** may include one or more batteries. These batteries may also be modular and replaceable. In other illustrative examples, energy system **620** may be a fuel cell or some other suitable type of energy system.

Energy harvesting system **622** is configured to generate power for components in autonomous vehicle **600** from the environment around autonomous vehicle **600.** For example, energy harvesting system **622** may include at least one of a biomechanical harvesting system, a piezoelectric harvesting system, a thermoelectric harvesting system, a tree-metabolic harvesting system, solar cells, a micro wind turbine generator, an ambient radio wave receiver, and other suitable types of energy harvesting systems that generate power from the environment around autonomous vehicle **600.**

In this illustrative example, controller **610** is associated with support structure **602.** As depicted, controller **610** takes the form of hardware and may include software.

Controller **610** is configured to control the operation of autonomous vehicle **600.** Controller **610** may provide level of intelligence **624.** Level of intelligence **624** may vary depending on the particular implementation of autonomous vehicle **600.** Level of intelligence **624** may be one example of level of intelligence **211** in **Figure 2****.**

In some cases, level of intelligence **624** may be such that controller **610** receives specific commands. These commands may include, for example, a direction of travel, a waypoint, when to generate information **220** using sensor system **606,** and other similar commands.

In other illustrative examples, level of intelligence **624** may be higher such that autonomous vehicle **600** may receive a task. Controller **610** may identify operations for performing the task. This task may be a fixed task in which autonomous vehicle **600** follows a path in a particular area to generate information **220** using sensor system **606.**

In other illustrative examples, level of intelligence **624** may be even higher such that autonomous vehicle **600** is configured to communicate with other autonomous vehicles to coordinate performing one or more tasks. For example, controller **610** may include a circuit, a computer program, an artificial intelligence system, and other suitable types of processes that may provide a desired level for level of intelligence **624.**

In these illustrative examples, intelligence system **628** may provide level of intelligence **624.** Intelligence system **628** may use an expert system, a neural network, fuzzy logic, or some other suitable type of system to provide level of intelligence **624.**

Level of intelligence **624** in controller **610** may allow for functions such as dynamic path planning. In this manner, obstacles may be identified along a path and may therefore be avoided. This identification and avoidance of obstacles may be performed in real time. These obstacles may include, for example, without limitation, a branch, a tree trunk, and other obstacles in forest **206.**

Controller **610** also may monitor health of different systems in autonomous vehicle **600.** For example, controller **610** may monitor a level of energy being provided or remaining in power source **612.** If power source **612** only includes batteries in energy system **620,** controller **610** may direct autonomous vehicle **600** to return to base for the recharging or exchange of batteries.

The illustration of autonomous vehicle **600** in **Figure 6** is not meant to imply limitations to the manner in which autonomous vehicle **600** may be implemented. In other illustrative examples, autonomous vehicle **600** may include other components in addition to or in place of the ones depicted. For example, autonomous vehicle **600** also may include systems for performing state changes. These systems may include, for example, without limitation, a tree logging system, a chemical dispersant system, a water distribution system, and other suitable types of systems.

In yet other illustrative examples, sensor system **606** may include a laser scanner used below the surface of a tree canopy to determine tree size. As another example, sensor system **606** may consist of soil moisture and nutrient monitoring probes that may be deployed to identify optimal timing and methods for planting. For example, these nutrient monitoring probes may be used to sample soil at various depths to determine the quantity of carbon or other elements within the soil of forest **206.** In still other illustrative examples, sensor system **606** may be used to sample water run-off, streams, and other bodies of water, such as body of water **129** in **Figure 1****,** to determine state changes **404** of these bodies of water within forest **206.**

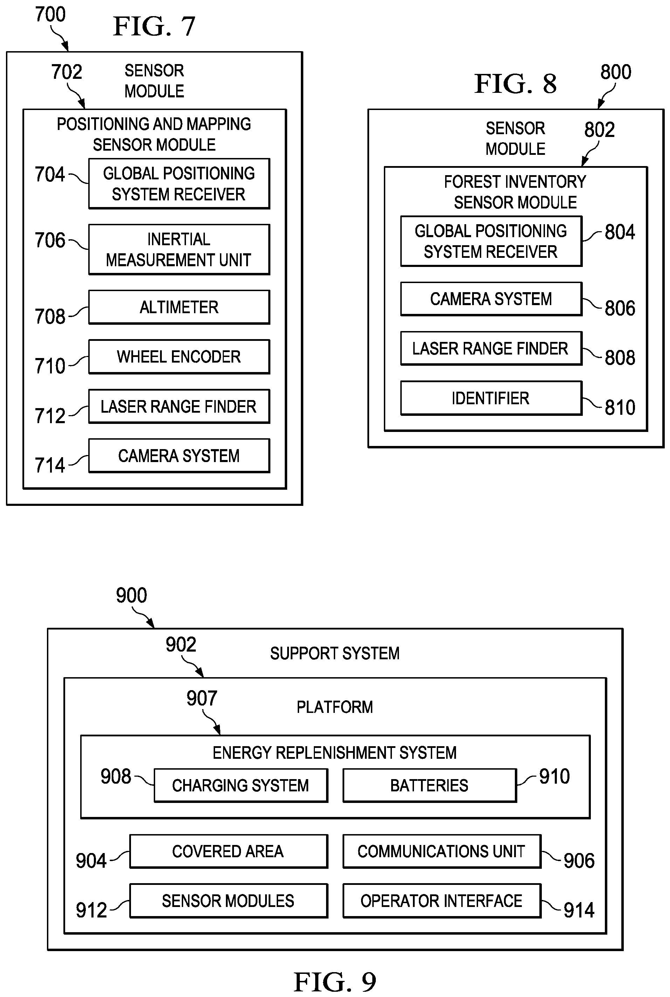

Turning now to **Figure 7****,** an illustration of a block diagram of a positioning and map building sensor module is depicted in accordance with an illustrative embodiment. As depicted, sensor module **700** is an example of one implementation of sensor module **616** in sensor system **606** in **Figure 6****.**

Sensor module **700** takes the form of positioning and mapping sensor module **702.** Positioning and mapping sensor module **702** may be removable or fixed within sensor system **606** depending on the particular implementation.

As depicted, sensor module **700** includes global positioning system receiver **704,** inertial measurement unit **706,** altimeter **708,** wheel encoder **710,** laser range finder **712,** and camera system **714.**

Global positioning system receiver **704** may be used to identify a location of the global positioning system receiver in autonomous vehicle **600** in three-dimensional coordinates. These coordinates may include latitude, longitude, and altitude. Global positioning system receiver **704** uses a satellite system to provide these three-dimensional coordinates.

Inertial measurement unit **706** may also be used to identify the three-dimensional coordinates of autonomous vehicle **600.** Inertial measurement unit **706** may supplement or provide refinement of positions generated by global positioning system receiver **704.**

As depicted, altimeter **708** may identify an altitude of autonomous vehicle **600** when global positioning system receiver **704** does not provide a desired level of accuracy. In these examples, wheel encoder **710** may provide an odometer reading. Specifically, wheel encoder **710** may estimate distance traveled by counting the number of rotations of the wheel.

In the illustrative examples, laser range finder **712** is configured to identify distances to different objects around autonomous vehicle **600.** Laser range finder **712** may generate three-dimensional coordinates for features around autonomous vehicle **600.** In particular, laser range finder **712** may generate data for a point cloud. This point cloud may be used to generate a three-dimensional map of one or more locations in forest **206.**

Camera system **714** is configured to generate images. These images may be correlated with data for the point cloud. In these illustrative examples, camera system **714** may include one or more cameras. For example, camera system **714** may include a visible light camera, a stereographic camera, an infrared camera, and other suitable types of cameras.

The illustration of sensor module **700** is not meant to imply limitations to the manner in which other sensor modules in sensor system **606** may be implemented for generating positioning and mapping information. For example, other sensor modules may exclude wheel encoder **710** and altimeter **708.** In still other illustrative examples, camera system **714** may be unnecessary.

In yet other illustrative examples, sensor module **700** may include a processor unit to pre-process information generated for mapping a location. Further, wheel encoder **710** may be used with ground-based vehicles and may be unnecessary with the aircraft or other vehicles.

Turning now to **Figure 8****,** an illustration of a block diagram of a sensor module is depicted in accordance with an illustrative embodiment. In this depicted example, sensor module **800** is another example of an implementation for sensor module **616** in sensor system **606** in **Figure 6****.** As depicted, sensor module **800** takes the form of forest inventory sensor module **802.**

In this illustrative example, forest inventory sensor module **802** includes a number of different components. For example, forest inventory sensor module **802** includes global positioning system receiver **804,** camera system **806,** laser range finder **808,** and identifier **810.**

Global positioning system receiver **804** is configured to identify a location of sensor module **800** and, in particular, the location of autonomous vehicle **600.** Camera system **806** is configured to generate images of the environment around autonomous vehicle **600.** In particular, these images may be images of trees and other vegetation.

Laser range finder **808** is configured to identify distances to various objects such as trees or other vegetation. Laser range finder **808** is configured to generate information about the location of these trees with respect to autonomous vehicle **600.**

Identifier **810** is configured to classify trees and plants in forest **206.** Identifier **810** may take the form of hardware and may include software. In these illustrative examples, identifier **810** may obtain images from camera system **806** and identify trees and vegetation based on the recognition of leaves, flowers, and other features that may be identified in the images.

Thus, the location of a particular tree or piece of vegetation may be identified knowing the location of autonomous vehicle **600** using information from global positioning system receiver **804.** In this manner, identifier **810** may perform some processing of position information to generate information about species of trees and other vegetation and the location of these species in forest **206.**

Although these illustrative examples depict forest inventory sensor module **802** with global positioning system receiver **804,** camera system **806,** laser range finder **808,** and identifier **810,** other components or sensors may be used in addition to or in place of the components illustrated in this figure. For example, sensors in forest inventory sensor module **802** may include hyperspectral imaging sensors, gas sensors, water quality sensors, airborne and terrestrial laser scanners, decay detectors, ground-penetrating radar, or other suitable types of sensors depending on the particular implementation.

With reference now to **Figure 9****,** an illustration of a block diagram of a support system is depicted in accordance with an illustrative embodiment. In this illustrative example, support system **900** is an example of components that may be used in a support system in support systems **213** in **Figure 2****.**

As depicted, support system **900** has a number of different components. Support system **900** includes platform **902,** covered area **904,** communications unit **906,** energy replenishment system **907,** sensor modules **912,** and operator interface **914.**

In this illustrative example, platform **902** is a structure on which autonomous vehicle **600** in **Figure 6** may land or move onto depending on the particular implementation. Platform **902** may be a mobile platform, a stationary platform, or some other suitable type of platform in these illustrative examples.

Covered area **904** may be an area in which autonomous vehicle **600** may be sheltered from the environment. Communications unit **906** may provide communications with autonomous vehicle **600,** forestry manager **202,** or some other suitable component.

Energy replenishment system **907** may include charging system **908,** batteries **910,** and other suitable components. Energy replenishment system **907** may be configured to recharge or otherwise provide energy system **620** in **Figure 6** with power.

Charging system **908** is configured to recharge energy system **620** in autonomous vehicle **600** in **Figure 6****.** Batteries **910** may be used to replace batteries in energy system **620** when batteries are used in energy system **620,** instead of recharging batteries depending on the condition of the batteries. Additionally, sensor modules **912** are examples of modules that may be replaceable in number of sensor modules **614** in **Figure 6****.**

Operator interface **914** may be a display system with a touch screen in these illustrative examples. Operator interface **914** may be viewed by personnel **138** in **Figure 1** to receive commands, missions, or other information about forest **206.** Operator interface **914** may also be used to input visual inspection results or other information that may be used by analyzer **306** to perform analysis **300** in **Figure 3****.**

The illustration of components in support system **900** in **Figure 9** is only shown as an example and is not meant to limit the manner in which other support systems may be implemented. For example, other support systems may omit communications unit **906.** In still other illustrative examples, a support system may include a storage device configured to store information generated by autonomous vehicle **600** or other platforms.

The illustration of forestry management environment **200** in **Figure 2** and the different components in **Figures 2-9** are not meant to imply physical or architectural limitations to the manner in which forestry management environment **200** and the different components may be implemented. Other components in addition to or in place of the ones illustrated may be used. Some components may be unnecessary. Also, the blocks are presented to illustrate some functional components. One or more of these blocks may be combined, divided, or combined and divided into different blocks when implemented in an illustrative embodiment.

Further, the different components shown in **Figure 1** may be combined with components in **Figures 2-9****,** used with components in **Figure 2-9****,** or a combination of the two. Additionally, some of the components in **Figure 1** may be illustrative examples of how components shown in block form in **Figures 2-9** can be implemented as physical structures.

For example, in some illustrative examples, manned vehicles **224** may be omitted from forestry management environment **200** in generating information **220** in **Figure 2****.** In still other illustrative examples, personnel **216** also may be unnecessary for generating information **220.** In yet other illustrative examples, support systems **213** may be omitted. In still other illustrative examples, forestry manager **202** may be located on one of vehicles **212** in these illustrative examples.

Moreover, although specific groupings of sensors are illustrated in support system **900** in **Figure 9** and sensor module **800** in **Figure 8****,** those sensors may be included in sensor system **606** without taking the form of a removable sensor module. In other words, sensor module **800** and support system **900** may be fixed in sensor system **606.**

The illustrative embodiments also recognize and take into account that collecting information from a forest using a forestry manager also may include collecting information for use in managing trees in a forest. For example, the information may be used to inform the process for planting and harvesting trees. For example, the information may be used to plant trees in the forest. In particular, the information may be used to plant seedlings in the forest.

Additionally, the information also may be used to in-fill portions of recently planted areas to establish a uniform coverage of healthy new-growth trees. In particular, the illustrative embodiments recognize and take into account that this information may be collected as part of forest inventory mission **408** in **Figure 4****.** In these illustrative examples, "in-fill" with respect to planting trees is the process of planting additional trees in an area that does not currently have the desired number, size, growth rate, health, or density of trees.

The illustrative embodiments recognize and take into account that the current methodologies for collecting information to plant trees may not be as accurate as desired. The illustrative embodiments also recognize and take into account that current methodologies for planting trees involve analyzing a history of weather conditions for a location in the forest. The history may be used along with forecasted weather conditions to plant trees in the forest. For example, this information may be used to determine when and where trees may be planted.

The illustrative embodiments recognize and take into account that the currently used methodologies for collecting information to plant trees do not provide as accurate of information as desired. Accurate information is needed for foresters to plant seedlings such that the seedlings grow as desired. When the soil is too cold for planting, seedlings may die or may not grow as desired. Further, drought conditions may also make planting seedlings more difficult than desired to obtain desired growth of the seedlings.

Without accurate information, foresters that would like to plant as early in the season as desired may risk the seedlings freezing or dying. As a result, foresters may utilize a more expensive planting strategy to minimize seedling mortality rates given uncertainties in soil conditions and weather conditions. For example, a forester may choose to plant more seedlings than required to account for future losses. However, planting a larger amount of seedlings may result in higher planting costs.

In other cases, a forester may choose to plant larger seedlings as measured by the root collar diameter, or choose to plant containerized seedlings. Both larger seedling stock and containerized seedling stock are more expensive to procure, and may not provide a desired increase in seedling health given favorable soil conditions and weather conditions. In other words, with favorable soil conditions and weather conditions, a less expensive bare root seedling may be as effective as a more expensive containerized seedling. Thus, knowing soil conditions and weather conditions prior to planting trees may allow foresters to more effectively plan planting processes than can be done with currently used methodologies.

The illustrative embodiments recognize and take into account that the information collected using an illustrative embodiment may be used to more accurately determine what types of seedlings and how many seedlings should be planted in addition to when and where seedlings should be planted. As a result, one or more illustrative embodiments may reduce the cost for planting trees.

The illustrative embodiments also recognize and take into account that collecting information needed to determine when forest operations should be performed may be more difficult than desired. These forest operations may include harvesting, inspecting, core sampling, controlling weeds, measuring, thinning, and other suitable types of operations. For example, the information for making harvesting decisions is currently collected by operators walking through the forest and taking measurements. These measurements include the height and diameter of trees. Height and diameter measurements may be used to determine whether an area of the forest is ready for harvesting. For example, when the diameter of trees in an area of the forest reaches a desired threshold value, trees in that area of the forest may be ready to harvest.

In other examples, soil data may be measured and used to determine whether the soil conditions are desired soil conditions for logging operations. As an illustrative example, personnel managing a forest may want to know soil conditions prior to performing harvesting operations to minimize erosion from the use of harvesting equipment. In this case, if the soil is wetter than desired, erosion of the top layer of soil may be increased with the use of harvesting equipment. This erosion of the top layer of soil may impact growth of reforested trees in these illustrative examples.

Further, wetter than desired soil may make it more difficult to operate harvesting equipment. As an example, harvesting equipment may get stuck in wet soil.

In other illustrative examples, drier than desired soil may cause an undesired amount of dust to be blown into the air during harvesting operations. This dust may also impact the top soil layer. In still other illustrative examples, soil conditions may aid in monitoring the risk of certain type of pests, depending on the particular implementation. Although some of these conditions may be estimated using weather forecasts and weather data, these sources of information may not identify the current condition of the soil conditions as accurately as desired.

Currently, trained operators may traverse areas that are difficult to access, hazardous to access, difficult to navigate, or some combination thereof. Many regulatory requirements also require at least two operators to minimize risks in collecting soil data. As a result, travel to and from different areas of a forest may result in a substantial labor cost. Further, the travel also may be costly with regard to equipment. The terrain may result in increased maintenance costs of the equipment that may be greater than desired.

The illustrative embodiments also recognize and take into account that when operators are obtaining soil samples, more than one sample may be needed in a particular area to account for elevation changes, land features, or other factors that may affect the density, moisture, chemical content, and other parameters of the soil in the locations. Thus, the cost for obtaining desired information about the soil in an area in which forest operations are planned may be greater than desired.

Further, if the area is not ready for forest operations, the collection of data is repeated again at a later time. As a result, the cost of obtaining information for forest operations may be greater and more difficult than desired.

Thus, the illustrative embodiments provide a method and apparatus for planting trees, monitoring trees, harvesting trees, or a combination thereof. In these illustrative examples, the information collected relates to a number of soil conditions in the forest.

In one illustrative embodiment, a forestry manager is configured to receive information relating to a number of soil conditions for a location in a forest from a sensor system deployed by a group of aerial vehicles. The forestry manager is also configured to identify a mission based on the number of soil conditions.

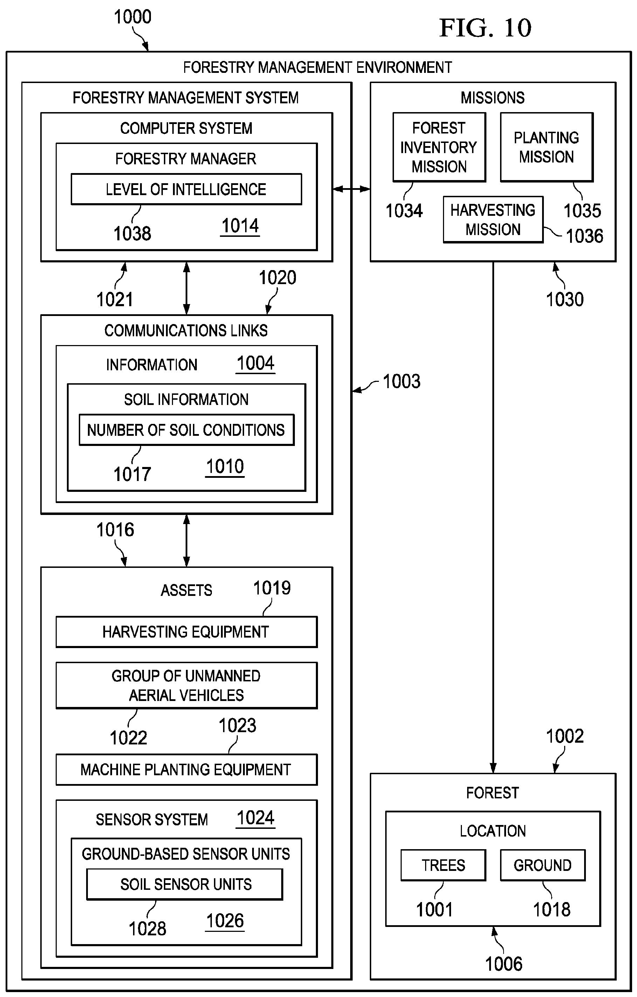

With reference now to **Figure 10****,** an illustration of a block diagram of a forestry management environment is depicted in accordance with an illustrative embodiment. In this illustrative example, forestry management environment **1000** is an environment in which planting, harvesting, or planting and harvesting of trees **1001** in forest **1002** may occur.

In this example, forestry management environment **1000** includes forestry management system **1003.** The components shown in forestry management system **1003** may be part of a forest sensor deployment and monitoring system in these illustrative examples.

As depicted, forestry management system **1003** in forestry management environment **1000** is configured to generate and analyze information **1004** about location **1006** in forest **1002.** In particular, information **1004** about location **1006** in forest **1002** takes the form of soil information **1010.** Information **1004** may be used to determine whether location **1006** in forest **1002** has favorable conditions for planting of trees **1001.** Additionally, information **1004** also may be used to determine whether trees **1001** in location **1006** in forest **1002** are ready for harvesting.

In these illustrative examples, forestry management system **1003** comprises forestry manager **1014** and assets **1016.** Assets **1016** are configured to generate information **1004** about location **1006** in forest **1002.** In these illustrative examples, soil information **1010** in information **1004** includes number of soil conditions **1017.**

In these illustrative examples, soil information **1010** about number of soil conditions **1017** may include moisture, a temperature, conductivity, nitrogen content, pH, calcium content, salt content, and a nutrient content, and other suitable soil conditions. Number of soil conditions **1017** may be used to determine when and where to plant trees **1001** in location **1006.** Number of soil conditions **1017** also may be used to determine what tree species, seedling type, or both tree species and seedling type for trees **1001** should be planted. For example, information about number of soil conditions **1017** in location **1006** may inform foresters that a certain species of tree may grow better in location **1006.**

In this example, a particular nutrient content or pH may be more favorable to a certain species of trees **1001.** In other illustrative examples, information about moisture in location **1006** may be used to select the type of seedling for trees **1001** that provides the desired density, growth, health, or other parameters for planting trees **1001** in location **1006.**

Further, number of soil conditions **1017** also may be used to determine whether forest operations may be performed on trees **1001.** In particular, number of soil conditions **1017** also may be used to determine whether ground **1018** in location **1006** is in a condition that is suitable for operating harvesting equipment. For example, harvesting equipment **1019** may include trucks, tree loggers, and other types of equipment. Number of soil conditions **1017** may be used to determine whether ground **1018** in location **1006** is stable enough to move harvesting equipment **1019** into location **1006** and perform harvesting of trees **1001** in location **1006** in forest **1002.**

Additionally, number of soil conditions **1017** may also provide information as to how planting of seedlings should occur. For example, number of soil conditions **1017** may be used to determine whether machine planting or hand planting of seedlings for trees **1001** is preferable.

Machine planting is a method of mechanically planting trees **1001** in location **1006** in forest **1002** using machine planting equipment **1023.** Depending on number of soil conditions **1017,** machine planting may increase the seedling survival rate. For example, when the soil in ground **1018** is hard and dry, a machine planter may break up the soil which may promote better root growth for seedlings.

Of course, the selection of the type of planting method may also depend on the availability of machine planting equipment **1023** and other suitable factors that may produce the highest survival rate of the seedlings for trees **1001** while lowering the cost of planting trees **1001** as compared to currently used methodologies. In other illustrative examples, if number of soil conditions **1017** are too wet for certain types of machine planting equipment, manual planting methods or other planting methods may be used, depending on the particular implementation.

In these illustrative examples, forestry manager **1014** is configured to receive information **1004** from assets **1016** over communications links **1020.** In these illustrative examples, communications links **1020** take the form of wireless communications links.

As depicted, forestry manager **1014** may be implemented using hardware, software, or some combination thereof. In particular, forestry manager **1014** may be implemented in computer system **1021.**

Assets **1016** include a group of aerial vehicles such as group of unmanned aerial vehicles **1022.** Assets **1016** also include sensor system **1024,** and harvesting equipment **1019.** In some illustrative examples, the group of aerial vehicles may be manned aerial vehicles.

Sensor system **1024** takes the form of ground-based sensor units **1026.** Ground-based sensor units **1026** may take the form of soil sensor units **1028.**

In this illustrative example, information **1004** may be generated by at least one of group of unmanned aerial vehicles **1022** and ground-based sensor units **1026** in sensor system **1024.** In this example, a ground-based sensor unit in number of ground-based sensor units **1026** is configured to generate information **1004** about at least one of location **1006** of the ground-based sensor unit, a trajectory of the ground-based sensor unit, and an orientation of the ground-based sensor unit. In this manner, ground-based sensor units **1026** provide information **1004** about ground-based sensor units **1026** and the environment around ground-based sensor units **1026.**

As depicted, ground-based sensor units **1026** may be deployed by group of unmanned aerial vehicles **1022.** In other words, group of unmanned aerial vehicles **1022** may drop ground-based sensor units **1026** such that ground-based sensor units **1026** land in location **1006** in forest **1002.** In particular, ground-based sensor units **1026** may be deployed to land on ground **1018** in forest **1002.**

In these illustrative examples, forestry manager **1014** is configured to analyze information **1004** about location **1006** in forest **1002** to determine where and how trees **1001** should be planted in location **1006.** Further, information **1004** also may be used to determine what types of trees **1001** should be planted. For example, information **1004** may be used to determine whether bare root seedlings or containerized seedlings should be planted in location **1006.** Additionally, information **1004** also may be used to determine different sizes of seedlings that may be used. The selection of the types and sizes of seedlings may be made to reduce the cost of planting trees **1001,** to increase the likelihood that the seedlings will survive, or some combination thereof.

Forestry manager **1014** also is configured to analyze information **1004** about location **1006** in forest **1002** to determine whether trees **1001** in location **1006** in forest **1002** are ready for harvesting. In particular, number of soil conditions **1017** may be used to determine whether ground **1018** is suitable for harvesting equipment **1019** to traverse location **1006.**

In these illustrative examples, forestry manager **1014** may identify at least one mission in missions **1030.** In these illustrative examples, the identification of the mission may be an identification of a mission to be performed without generating a number of tasks to perform the mission. In other illustrative examples, the identification of the mission may include generating the number of tasks for the mission. The identification of the mission also may include identifying and assigning assets **1016** to perform particular tasks in the mission.

In these illustrative examples, missions **1030** may include at least one of forest inventory mission **1034,** planting mission **1035,** harvesting mission **1036,** and other suitable types of missions. As depicted, forest inventory mission **1034** is configured to generate information **1004** which includes soil information **1010** in these illustrative examples. Planting mission **1035** is configured to plant trees **1001** in forest **1002.** Harvesting mission **1036** is configured to harvest trees **1001** in forest **1002.** In these illustrative examples, forestry manager **1014** may have level of intelligence **1038** that is configured to control the operation of assets **1016** without requiring input from a human operator.

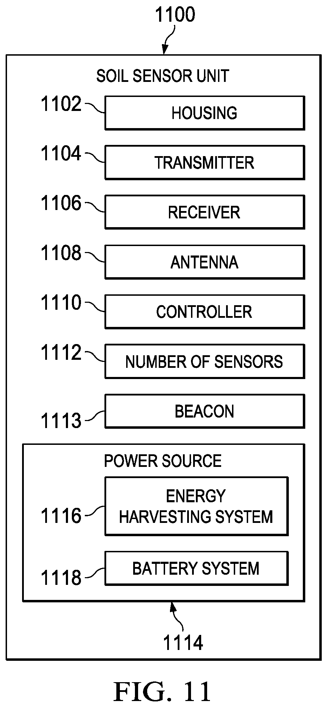

Turning now to **Figure 11****,** an illustration of a block diagram of a ground-based sensor unit is depicted in accordance with an illustrative embodiment. In this depicted example, soil sensor unit **1100** is an example of a soil sensor in soil sensor units **1028** for ground-based sensor units **1026** in **Figure 10****.**

As depicted, soil sensor unit **1100** includes a number of different components. In this illustrative example, soil sensor unit **1100** comprises housing **1102,** transmitter **1104,** receiver **1106,** antenna **1108,** controller **1110,** number of sensors **1112,** beacon **1113,** and power source **1114.**

Housing **1102** is a structure configured to support or hold the other components in soil sensor unit **1100.** Housing **1102** may be comprised of a number of different types of materials. For example, housing **1102** may be comprised of at least one of plastic, metal, a composite material, a biodegradable material, biodegradable closed cell extruded polystyrene foam, polycarbonate, and other suitable types of materials.

The type of material selected for housing **1102** may depend on whether soil sensor unit **1100** is a disposable sensor unit or a recoverable sensor unit. If soil sensor unit **1100** is a disposable sensor unit, the materials selected may be based on cost, biodegradability, or some combination thereof. If soil sensor unit **1100** is selected to be a recoverable sensor unit, the materials may be selected for durability.

In these illustrative examples, transmitter **1104** is configured to transmit information over antenna **1108.** Receiver **1106** is configured to receive information over antenna **1108.** In some illustrative examples, transmitter **1104** and receiver **1106** may be a single component such as a transceiver.

Controller **1110** is implemented using hardware and may include software. Controller **1110** may take various forms depending on the particular implementation. For example, controller **1110** may include at least one of a processor unit, an application specific integrated circuit, a digital signal processor, or some other suitable type of hardware.

As depicted, controller **1110** is configured to control the operation of components in soil sensor unit **1100.** For example, controller **1110** may control the generation of information by number of sensors **1112,** the transmitting and receiving of information by transmitter **1104** and receiver **1106,** and other suitable operations.

In these illustrative examples, number of sensors **1112** is configured to generate information about the soil. This information may be processed by controller **1110** prior to being transmitted to a remote location through transmitter **1104** over antenna **1108.**

In these illustrative examples, number of sensors **1112** may include at least one of a temperature sensor, a moisture sensor, a pH sensor, an electroconductivity sensor, a global positioning system receiver, a nitrate sensor, a calcium sensor, and other suitable types of sensors. Depending on the configuration of soil sensor unit **1100,** number of sensors **1112** may include a soil temperature sensor and moisture sensor. Other sensors also may be included in other configurations of soil sensor unit **1100.**

The different sensors in number of sensors **1112** may be implemented using a number of currently available sensors. Examples of implementations for a moisture sensor include a frequency domain capacitive probe, a frequency domain reflectometry sensor, a phase transmission sensor, an amplitude domain reflectometry sensor, a time domain reflectometry sensor, a time domain transmissionmetry sensor, a soil tensiometer, a rhizon soil moisture sampler, a gravimetric soil moisture measurement sensor, a heat dissipation moisture sensor, a soil psychrometer, a resistive probe, a gypsum block sensor, a resistance block sensor, a granular matrix sensor, a neutron probe, and other suitable types of sensors.

In these illustrative examples, the type of sensor for number of sensors **1112** may be selected based on whether the sensor unit is a disposable sensor unit or a recoverable sensor unit. With a disposable sensor unit, a simple resistive probe may be used to detect moisture in the soil. Conversely, a more expensive frequency domain reflectometry sensor may be used when the sensor unit is a recoverable sensor. Of course, other types of moisture sensors may be used when number of sensors **1112** are disposable sensors or recoverable sensors depending on the particular implementation.

Moreover, the type of sensor used for number of sensors **1112** may be determined by the type of assets **1016** used to deploy the sensor. For example, for small unmanned aerial vehicles in group of unmanned aerial vehicles **1022** which may have smaller load capacities, a light-weight sensor may be used. In other cases, a larger sensor, such as a time domain reflectometer, may be deployed from a ground vehicle in assets **1016,** depending on the particular implementation.

Further, number of sensors **1112** also may include one or more sensors to determine whether soil sensor unit **1100** has been deployed in a desired manner. For example, number of sensors **1112** may include an accelerometer or other devices configured to identify the orientation of soil sensor unit **1100.** In yet other illustrative examples, a sensor used to generate soil information also may be used to determine whether soil sensor unit **1100** is deployed as desired. For example, a moisture sensor may be used to determine whether readings of moisture indicate that the moisture sensor has been embedded or has penetrated the ground.

As depicted, beacon **1113** is configured for use in recovering soil sensor unit **1100.** Beacon **1113** may be an attention gathering device such as a light source or a sound source to attract the attention of a human operator. In other illustrative examples, beacon **1113** may be a radio frequency transmitter configured to transmit signals that may be used to locate soil sensor unit **1100.**

In these illustrative examples, power source **1114** is configured to generate power used to operate the different components in soil sensor unit **1100.** For example, power source **1114** may provide power to transmitter **1104,** receiver **1106,** controller **1110,** and number of sensors **1112.**

Power source **1114** may take a number of different forms. For example, power source **1114** may include at least one of energy harvesting system **1116** and battery system **1118.** Energy harvesting system **1116** may be used to increase the operational life of soil sensor unit **1100.** Energy harvesting system **1116** may take a number of different forms similar to those described for energy harvesting system **622** in autonomous vehicle **600** in **Figure 6****.** For example, energy harvesting system **1116** may include at least one of a solar energy harvester, a thermoelectric ambient energy harvester, an ambient radio frequency (RF) harvester, a soil bioelectrochemical system (BES), a microwind generator, and other suitable types of energy harvesting devices.

Battery system **1118** may be comprised of one or more batteries. When used in conjunction with energy harvesting system **1116,** battery system **1118** may be recharged by energy harvesting system **1116.** Battery system **1118** may include a number of batteries. The type of battery selected may depend on whether soil sensor unit **1100** is configured to be disposable or recoverable. For example, if soil sensor unit **1100** is configured to be disposable, the battery may be selected based on cost and lowering the environmental impact on the location in which the soil sensor unit is used. As an example, a low self-discharge nickel-metal hydride (NiMH) battery may be used.

If soil sensor unit **1100** is configured to be recoverable, the performance of the battery may be used as criteria for selection. For example, the battery may be a thin film battery, a super capacity energy storage device, a lithium ion battery, or some other suitable type of battery.

The selection of components for soil sensor unit **1100** may vary depending on the goals for soil sensor unit **1100.** For example, if soil sensor unit **1100** is meant to be a disposable unit, the components may be selected to be as low cost as possible. For example, receiver **1106** may be omitted. As another example, housing **1102** may be selected to include a biodegradable material. With this type of implementation, soil sensor unit **1100** may only include a moisture sensor and a temperature sensor and other components in soil sensor unit **1100** may be omitted.

In other illustrative examples, soil sensor unit **1100** may be designed to be recoverable. When soil sensor unit **1100** is designed to be recoverable, soil sensor unit **1100** may include more components and may be designed to include components for use in locating soil sensor unit **1100** for recovery. For example, number of sensors **1112** may include a global positioning system receiver that generates information about the location of soil sensor unit **1100.** This location information may be used to recover soil sensor unit **1100.** For example, when soil sensor unit **1100** is recoverable, number of sensors **1112** may include more expensive and more sophisticated sensors. Number of sensors **1112** may include, for example, without limitation, a pH sensor, a nitrogen sensor, and other suitable types of sensors to obtain additional information about the soil.

The illustration of forestry management environment **1000** and the different components in forestry management environment **1000** in **Figure 10** and **Figure 11** are not meant to imply limitations to the manner in which an illustrative embodiment may be implemented. For example, in some illustrative examples, soil sensor unit **1100** in **Figure 11** may only include transmitter **1104** and not receiver **1106.**

As another illustrative example, assets **1016** in forestry management system **1003** may include other components for generating information **1004.** For example, a group of unmanned ground vehicles also may be used in assets **1016** to generate information **1004** about whether location **1006** in forest **1002** is ready for harvesting of trees **1001.**

For example, although not shown in assets **1016,** assets **1016** also may include planting equipment. The planting equipment may be used to plant trees **1001.** In particular, the planting equipment may be used to plant trees **1001** in the form of seedlings.

As another illustrative example, information **1004** may be analyzed by forestry management system **1003** to determine whether undesired conditions are present in location **1006** in forest **1002.** For example, number of soil conditions **1017** may indicate that conditions may be present that may result in a forest fire starting in or around location **1006.** This identification may be used to initiate a warning mission in missions **1030.**

In still another illustrative example, transmitter **1104** and receiver **1106** may be implemented as a single component in the form of a transceiver. In yet other illustrative examples, sensor system **1024** may include other devices other than ground-based sensor units **1026.** For example, sensor system **1024** also may include a base station configured to receive information **1004** from ground-based sensor units **1026** and transmit information **1004** to forestry manager **1014.** In this illustrative example, the base station may be powered by an energy harvesting system such as a solar power generation system.

As yet another example, soil sensor unit **1100** may be implemented using modules. For example, when soil sensor unit **1100** is a recoverable soil sensor unit, soil sensor unit **1100** may have a module similar to sensor module **616 in** **Figure** 6 that may be replaceable.

In yet other illustrative examples, soil sensor unit **1100** may include other components not shown in **Figure 11****.** For example, soil sensor unit **1100** also may include a logic circuit, a regulator, a printed circuit board, an input/output interface, a display, and other suitable components depending on the particular implementation.

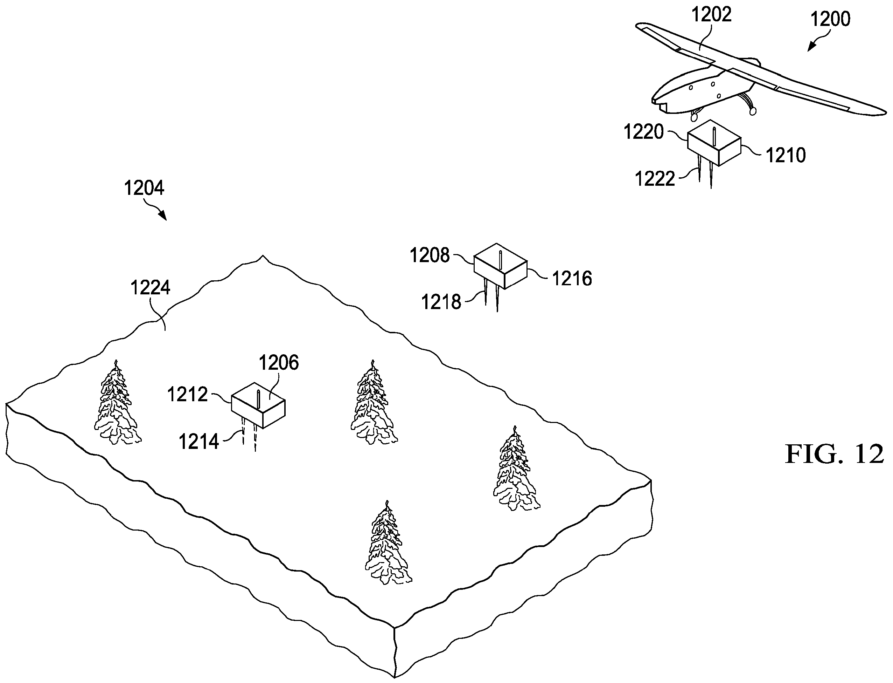

Turning now to **Figure 12****,** an illustration of a deployment of a sensor system for obtaining soil information is depicted in accordance with an illustrative embodiment. In this illustrative example, forest area **1200** is an example of location **1006** in forest **1002** in **Figure 10****.** As depicted, forest area **1200** is an open location in which trees are absent. Reforestation is desirable for this particular location and soil information may be obtained to determine when and how planting of trees, such as trees **1001** in **Figure 10****,** should occur in forest area **1200.**

In this illustrative example, unmanned aerial vehicle **1202** is configured to deploy soil sensor units **1204** in a sensor system. Unmanned aerial vehicle **1202** may be one of group of unmanned aerial vehicles **1022** in **Figure 10****.** The unmanned aerial vehicles collecting information generated by soil sensor units **1204** may be the same or different than the unmanned aerial vehicles that deployed soil sensor units **1204** in these illustrative examples.

As depicted, soil sensor units **1204** include soil sensor unit **1206,** soil sensor unit **1208,** and soil sensor unit **1210.** Of course, many other sensor units may be present but not shown in this particular example.

Unmanned aerial vehicle **1202** deploys soil sensor units **1204** through an airdrop operation in which unmanned aerial vehicle **1202** drops soil sensor units **1204** while flying over forest area **1200.** Soil sensor units **1204** may provide location information to unmanned aerial vehicle 1202 or other devices.

With the use of disposable sensors for soil sensor units **1204,** a global positioning system transmitter may be omitted to reduce the size, weight, and cost of the soil sensor units **1204.** In this case, the location of a sensor in soil sensor units **1204** may be determined using a personal identification number or an identification code. For example, each sensor in soil sensor units **1204** may be assigned a personal identification number or identification code. The identification may be recorded on the sensor using a radio-frequency identification tag attached to the sensor at time of manufacture. As the sensor is deployed, the identification is read and associated with the global positioning system coordinates of unmanned aerial vehicle **1202** at the time of the drop.

Based on the location of unmanned aerial vehicle **1202,** the velocity at which the sensor is dropped, and the altitude at which the sensor is dropped, a location of the sensor may be estimated. In this manner, the location of the sensor may be estimated and recorded with reasonable precision without the need to add costly components to a disposable sensor. As a result, when unmanned aerial vehicle **1202** or other unmanned aerial vehicles collect data from soil sensor units **1204,** unmanned aerial vehicle **1202** may know the location of sensors in soil sensor units **1204** with enough precision to wirelessly receive information from the sensors.

Recoverable soil sensor units **1204,** on the other hand, may identify location information using other components. For example, as unmanned aerial vehicle **1202** deploys soil sensor units **1204,** soil sensor units **1204** are turned on. Of course, soil sensor units **1204** may be turned on at any time including before or after deployment by unmanned aerial vehicle **1202.**

Soil sensor units **1204** may transmit location information and identification information such that the location of each sensor unit can be identified. In these illustrative examples, a location may be in two-dimensional or three-dimensional coordinates depending on the particular implementation. For example, the location may be in latitude and longitude and also may include an altitude. Recoverable soil sensor units **1204** may include a global positioning system receiver in these illustrative examples.

In other illustrative examples, soil sensor units **1204** may include transmitters and not employ global positioning system receivers. Instead, soil sensor units **1204** may include radio frequency identifier tags that are configured to transmit identifiers. The coordinates of the aircraft dropping soil sensor units **1204** may be associated with the identifiers to obtain an approximate location of soil sensor units **1204.**

In this example, soil sensor unit **1206** has housing **1212** and pins **1214.** Soil sensor unit **1208** has housing **1216** and pins **1218.** Soil sensor unit **1210** has housing **1220** and pins **1222.** The pins and the housings are weighted such that the pins will fall and penetrate ground **1224** when soil sensor units **1204** reach the ground. In other words, soil sensor units **1204** are bottom heavy.

In this illustrative example, the distribution of soil sensor units **1204** may vary depending on the particular implementation. For example, soil sensor units **1204** may be deployed such that about one mile, about ten miles, or some other suitable distance is present between soil sensor units **1204.** Soil sensor units **1204** may be dropped in various patterns such as a grid, a spiral, or some other suitable pattern.

In deploying soil sensor units **1204,** the distance between soil sensor units **1204** may depend on the terrain of ground **1224.** For example, with varying terrain such as high hills, a microclimate may be produce in a portion of the high hills. A microclimate is a local atmospheric zone where the climate may differ from surrounding areas.

As an example, one side of a hill may receive more rain than the other side. In this case, soil sensor units **1204** may be deployed closer together to take into account these microclimates. In another illustrative example where flat land is present, fewer soil sensor units **1204** may be deployed, soil sensor units **1204** may be deployed at larger intervals, or both.

In other illustrative examples, soil sensor units **1204** may be deployed based on the type of soil in ground **1224.** For example, more soil sensor units **1204** may be desired in an area with soft soil than in an area with clay or rock. Moreover, soil sensor units **1204** may not be deployed in areas where streams, rivers, lakes, roads, and other features are present, depending on the particular implementation.

In still other illustrative examples, the distance between soil sensor units **1204** may depend on the desired granularity of information. For example, if a higher granularity of information is desired, more of soil sensor units **1204** may be deployed, soil sensor units **1204** may be deployed closer together, or a combination thereof.

After soil sensor units **1204** have been deployed, the soil sensor units may generate information about the soil in forest area **1200** and about other conditions in forest area **1200.** For example, soil sensor units **1204** may generate information about air temperature, humidity, and other conditions in addition to conditions about the soil in ground **1224** in forest area **1200.**

In this illustrative example, when soil sensor units **1204** are disposable, soil sensor units **1204** may be configured to transmit this information during selected periods of time. In this example, unmanned aerial vehicle **1202** or another unmanned aerial vehicle may fly over forest area **1200** to collect information generated by soil sensor units **1204** during those selected periods of time. As an example, soil sensor units **1204** may be programmed to transmit on preselected days and times. Unmanned aerial vehicle **1202** or other unmanned aerial vehicles may be programmed with the same schedule and may fly over soil sensor units **1204** during these preselected days and times.

The selection of times for transmission of information from soil sensor units **1204** may be determined by the flight pattern of unmanned aerial vehicle **1202** in these illustrative examples. For example, each of soil sensor units **1204** may stage transmission based on the distance between each sensor and the time it takes unmanned aerial vehicle **1202** to fly between soil sensor units in soil sensor units **1204.** In this manner, transmission time and energy consumption may be minimized when transmitting information from soil sensor units **1204.**

When soil sensor units **1204** are recoverable sensors, other components may be included in soil sensor units **1204.** For example, soil sensor units **1204** may be equipped with a receiver. In this case, unmanned aerial vehicle **1202** may transmit a signal to "wake up" the sensors. When a sensor in soil sensor units **1204** receives the wireless order to transmit, the sensor may then respond by transmitting a data log of soil sensor measurements to unmanned aerial vehicle **1202.**

As depicted, unmanned aerial vehicle **1202** may be various sizes depending on the weight and number of units present in soil sensor units **1204.** For example, if each sensor unit in soil sensor units **1204** weighs about 100 grams, then fifty sensor units in soil sensor units **1204** may weigh about 5 kilograms. With this size payload, unmanned aerial vehicle **1202** may be a small to medium-sized unmanned aerial vehicle. For example, a small unmanned aerial vehicle may be about four feet in length with about a ten foot long wingspan. In other examples, a medium-sized unmanned aerial vehicle may be about 35 feet in length with about a 36 foot rotor diameter. In still other illustrative examples, a medium-sized unmanned aerial vehicle may be about 26 feet in length with about a 44 foot wingspan. Of course, other combinations of lengths, wingspans or rotor diameters may be used for small and medium-sized unmanned aerial vehicles, depending on the functionality involved.

Although unmanned aerial vehicle **1202** is shown as a fixed wing aerial vehicle in these illustrative examples, rotorcraft also may be used to implement unmanned aerial vehicle **1202.** Of course, other numbers of one or more additional unmanned aerial vehicles may be used in addition to unmanned aerial vehicle **1202** to deploy soil sensor units **1204** in forest area **1200.**

In other illustrative examples, other types of assets **1016** may be used to deploy soil sensor units **1204** and receive information from soil sensor units **1204.** For example, a manned aerial vehicle may deploy soil sensor units **1204** when it is desired to drop a larger number of soil sensors units **1204** at one time. In another illustrative example, a ground vehicle may receive information from one or more soil sensor units **1204** in ground **1224.**

With the use of unmanned aerial vehicle **1202** to deploy soil sensor units **1204,** cost of deployment of soil sensor units **1204** over large areas may be reduced. In other words, deployment and data collection about locations to be reforested may be made by soil sensor units **1204** more quickly, easily, and with lower cost than currently used methods.

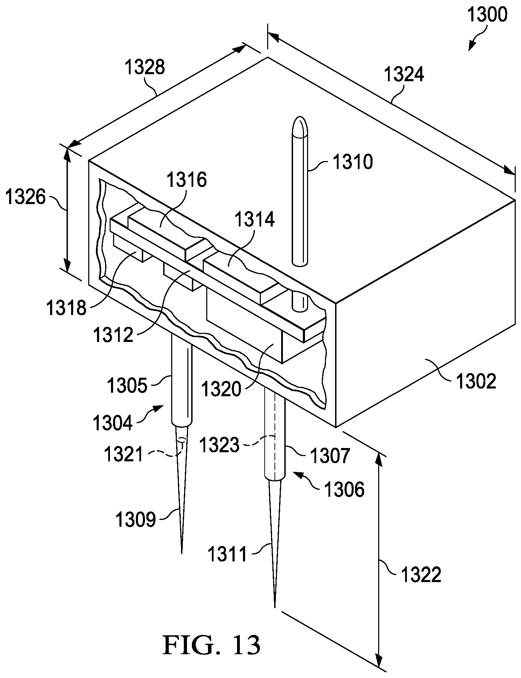

Turning now to **Figure 13****,** an illustration of a soil sensor unit is depicted in accordance with an illustrative embodiment. Soil sensor unit **1300** is an example of a physical implementation of soil sensor unit **1100** shown in block form in **Figure 11****.** Further, soil sensor unit **1300** may be used to implement one or more of soil sensor units **1204** in **Figure 12****.** More specifically, soil sensor unit **1300** may be an example of a disposable sensor unit. In other words, soil sensor unit **1300** is configured to be deployed and not retrieved in this illustrative example.

As depicted, soil sensor unit **1300** has housing **1302.** Housing **1302** is configured to provide a structure to be associated with components in soil sensor unit **1300.** In particular, other components may be associated with soil sensor unit **1300** by being contained in, connected to, or formed as part of housing **1302.** Materials used for housing **1302** may be selected based on lowering the cost for soil sensor unit **1300.** Further, housing **1302** may be comprised of a material that is biodegradable in these illustrative examples.

Soil sensor unit **1300** includes pin **1304** and pin **1306.** As depicted, pin **1304** and pin **1306** are metal pins. Sensors may be associated with or formed as part of pin **1304** or pin **1306.** In this illustrative example, pin **1304** and pin **1306** may function as a probe for moisture detection.

In this illustrative example, the weight of pin **1304** and pin **1306** relative to housing **1302** and the other components associated with housing **1302** is selected such that soil sensor unit **1300** is bottom heavy. In other words, soil sensor unit **1300** is configured to land with pin **1304** and pin **1306** pointing to and penetrating the ground such that pin **1304** and pin **1306** extend into the ground when soil sensor unit **1300** is deployed through an airdrop. Of course, soil sensor unit **1300** also may be deployed using a ground vehicle such as an unmanned ground vehicle that plants soil sensor unit **1300** into the ground.

Further, soil sensor unit **1300** includes antenna **1310** which is connected to printed circuit board **1312** seen inside of housing **1302** in this exposed view of housing **1302.** Transmitter **1314,** controller **1316,** and logic circuits **1318** also are connected to printed circuit board **1312.**

Transmitter **1314** is configured to transmit information using wireless communications links through antenna **1310.** Controller **1316** may be, for example, a microcontroller. Controller **1316** may control the operation of soil sensor unit **1300** in collecting and transmitting information about the soil. Logic circuits **1318** may detect signals from a moisture sensor that may be implemented using pin **1304,** pin **1306,** or both pin **1304** and pin **1306** to generate information in a form suitable for transmission. In these illustrative examples, pin **1304** and pin **1306** may be comprised of metal, and moisture may be determined based on a measurement of the resistance between pin **1304** and pin **1306.** Further, logic circuits **1318** also may include storage, memory, or other devices to temporarily store the information prior to transmission.

In some illustrative examples, pin **1304** and pin **1306** may have insulated portion **1305** and insulated portion **1307,** respectively. Insulated portion **1305** and insulated portion **1307** are configured to provide a desired level of accuracy for a measurement of the resistance between pin **1304** and pin **1306** at a desired depth under the surface of the ground. For example, insulated portion **1305** and insulated portion **1307** result in pin **1304** and pin **1306** having exposed portion **1309** and exposed portion **1311,** respectively. With insulated portion **1305** and insulated portion **1307** present, a measure of resistance may be made at only one depth instead of along the entire length of pin **1304** and pin **1306.** As a result, measurement of resistance between pin **1304** and **1306** may be localized at a desired depth under the surface of the ground.

In other words, interference from other measurements of resistance between pin **1304** and pin **1306** along the length of pin **1304** and pin **1306** may be prevented by insulated portion **1305** and insulated portion **1307** in these illustrative examples. Thus, measurement of the resistance may be specific to a particular depth and may be more accurate than if insulated portion **1305** of pin **1304** and insulated portion **1307** of pin **1306** are absent.

Additionally, pin **1304** and pin **1306** may have exposed portion **1309** and exposed portion **1311,** respectively. Exposed portion **1309** and exposed portion **1311** are configured to allow measurement of resistance between pin **1304** and pin **1306** at a desired depth underneath the soil. This depth may be predetermined by the type of soil or other suitable parameters. For example, a measure of resistance between pin **1304** and pin **1306** may be taken at the level of point **1321** in this illustrative example. This resistance measurement is used to determine the moisture content in the soil.

In this illustrative example, temperature sensor **1323** is also present in pin **1306.** Temperature sensor **1323** may be a thermocouple wire in this illustrative example. Temperature sensor **1323** is insulated by insulated portion **1307** of pin **1306.**

Temperature sensor **1323** helps provide a more accurate reading of moisture content in the soil as compared to only using resistance measurements. For example, when sun heats the soil and the soil warms up, the soil resistance changes. In this case, a false "dry" reading may occur from the resistance measurement between pin **1304** and pin **1306.** With the use of temperature sensor **1323,** soil sensor unit **1300** can correct the measurement to account for change in temperature in these illustrative examples. Of course, other types of temperature sensors may be used other than a thermocouple wire, depending on the particular implementation.

In these illustrative examples, the moisture sensor implemented using pin **1304,** pin **1306,** or both pin **1304** and pin **1306** may be configured based on soil type. For example, the moisture sensor may be calibrated based on information about soil type from previous soil survey missions. Because soil electrical resistance is a function of soil moisture content, soil temperature, and soil type, calibration of the moisture sensor aids soil sensor unit **1300** in providing more accurate information about soil electrical resistance.

Battery **1320** is connected to printed circuit board **1312.** Battery **1320** is configured to provide power to the different components in soil sensor unit **1300.**

In this illustrative example, pin **1304** and pin **1306** have length **1322.** Length **1322** may vary depending on the particular implementation. In one illustrative example, length **1322** may be about 10 centimeters. For example, measurements may be made into the soil up to about 10 centimeters in the ground when soil sensor unit **1300** is deployed.

In this particular example, housing **1302** of soil sensor unit **1300** has length **1324,** height **1326,** and depth **1328.** Length **1324** may be about 5 centimeters, height **1326** may be about 5 centimeters, and depth **1328** may be about 5 centimeters. Of course, in other illustrative examples, housing **1302** may have other dimensions or other shapes. In one illustrative example, housing **1302** may have a shape selected from one of a pyramid, a cube, or some other suitable shape other than the cuboid shown for housing **1302** in this depicted example.

Of course, the illustration of soil sensor unit **1300 in** **Figure 13** is not meant to imply limitations to the manner in which different soil sensor units may be implemented. For example, in other illustrative examples, soil sensor unit **1300** also may include a receiver. Additionally, soil sensor unit **1300** also may be implemented to include an energy harvesting device in addition to or in place of battery **1320.**

In still other illustrative examples, other numbers of pins may be used in addition to or in place of pin **1304** and pin **1306.** For example, a single pin, three pins, seven pins, or some other number of pins may be used depending on the particular implementation. The particular components selected for soil sensor unit **1300** may be based on cost, biodegradability, or some combination thereof when soil sensor unit **1300** is a disposable sensor unit.

Thus, soil sensor unit **1300** may result in more accurate information about moisture content of the soil. With the use of pin **1304,** pin **1306,** and temperature sensor **1323,** below the surface moisture content may be measured. As a result, rapidly changing surface conditions do not affect the accuracy of soil sensor unit **1300** when measuring soil conditions below the surface of the ground. These rapidly changing surface conditions may be, for example, at least one of dew, light rainfall, evaporation, and other surface conditions

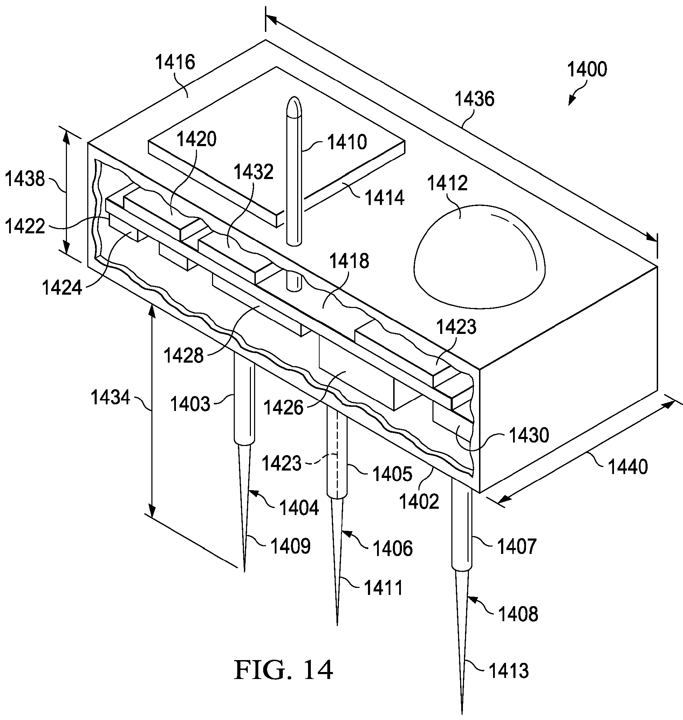

Turning now to **Figure 14****,** an illustration of a soil sensor unit is depicted in accordance with an illustrative embodiment. Soil sensor unit **1400** is an example of a physical implementation of soil sensor unit **1100** shown in block form in **Figure 11****.** Further, soil sensor unit **1400** may be used to implement one or more of soil sensor units **1204** in **Figure 12****.** More specifically, soil sensor unit **1400** may be an example of a recoverable sensor unit. In other words, soil sensor unit **1400** is configured to be retrieved at a later time. For example, soil sensor unit **1400** may be retrieved when planting of trees occurs.

As depicted, soil sensor unit **1400** has housing **1402.** Housing **1402** is configured to provide a structure to be associated with components in soil sensor unit **1400.** Materials used for housing **1402** may be selected based on durability for soil sensor unit **1400.** Further, housing **1402** may be comprised of at least one of metal, a plastic, aluminum, polycarbonate, polyvinyl chloride, and other suitable types of materials.

Soil sensor unit **1400** includes pin **1404** and pin **1406.** As depicted, pin **1404** and pin **1406** are metal pins. Sensors may be associated with or formed as part of pin **1404** or pin **1406.** In this illustrative example, pin **1404** and pin **1406** may function as a probe for moisture detection. Additionally, pin **1408** is associated with pin **1404** and may generate information about the soil.

As depicted, pin **1404,** pin **1406,** and pin **1408** have insulated portion **1403,** insulated portion **1405,** and insulated portion **1407,** respectively. Insulated portion **1403,** insulated portion **1405,** and insulated portion **1407** may provide for more accurate measurements of resistance between any two of pin **1404,** pin **1406,** and pin **1408** at a desired depth under the surface of the ground.

Additionally, pin **1404,** pin **1406,** and pin **1408** have exposed portion **1409,** exposed portion **1411,** and exposed portion **1413,** respectively. Resistance between two exposed portions of the pins in soil sensor unit **1400** may be used to determine the moisture level of the soil.

Temperature sensor **1423** may also be included in pin **1406** in these illustrative examples. Temperature sensor **1423** may be a thermocouple wire and may provide temperature information for soil sensor unit **1400.**

In these illustrative examples, sensors may be associated with at least one of pin **1404,** pin **1406,** and pin **1408** to generate soil information about soil in the ground when soil sensor unit **1400** is deployed. For example, the sensors may include at least one of a moisture sensor, a temperature sensor, a pH sensor, a nitrogen and nutrient content sensor, a salt content sensor, and other suitable types of sensors. As depicted, the weight of pin **1404** and pin **1406** relative to housing **1402** and the other components associated with housing **1402** is selected such that soil sensor unit **1400** is bottom heavy for deployment in an airdrop.

In this example, antenna **1410,** beacon **1412,** and solar cell **1414** are seen on exterior surface **1416** of housing **1402.** Solar cell **1414** is an example of an energy harvesting device that may be used to provide power to components in soil sensor unit **1400.**

Beacon **1412** may be configured to assist in recovery of soil sensor unit **1400.** Beacon **1412** may be, for example, at least one of a light emitting diode, a speaker, and other suitable types of attention attraction devices for human operators.

As can be seen in this exposed view of housing **1402,** soil sensor unit **1400** also includes a number of different components. Printed circuit board **1418** provides a structure for a number of different components within housing **1402.** Additionally, printed circuit board **1418** may also provide for electrical communication between different components in soil sensor unit **1400.** In this illustrative example, microcontroller **1420,** logic circuits **1422,** global positioning system receiver and antenna **1410,** power regulator **1424,** battery **1426,** energy harvesting circuit **1428,** input/output interface **1430,** and transceiver **1432** are connected to printed circuit board **1418.** Additionally, antenna **1410,** beacon **1412,** solar cell **1414,** pin **1404,** pin **1406,** and pin **1408** also are connected to printed circuit board **1418.**

In this illustrative example, energy harvesting circuit **1428** is configured to manage power generated by solar cell **1414.** Power regulator **1424** is configured to control the storage of power in battery **1426** and the distribution of power to different components in soil sensor unit **1400.** Further, in this illustrative example, transceiver **1432** also allows the receipt of signals in addition to transmitting signals. These signals may be exchanged with at least one of an unmanned aerial vehicle, a control station, another soil sensor unit, and other suitable types of devices. Thus, in contrast to soil sensor unit **1300,** soil sensor unit **1400** also may receive requests, data, commands, and other information for use in generating information about the soil.

The illustration of soil sensor unit **1400** is not meant to imply physical or architectural limitations to the manner in which an illustrative embodiment may be implemented. For example, although three pins are illustrated for soil sensor unit **1400,** fewer or more pins may be used.

In yet other illustrative examples, beacon **1412** may be omitted. In this implementation, location information generated by global positioning system receiver and antenna **1410** may be used to locate and recover soil sensor unit **1400.** As another illustrative example, although this example employs solar cell **1414,** other types of energy harvesting devices may be used in addition to or in place of solar cell **1414** to improve the operational life of soil sensor unit **1400.**

In this illustrative example, pin **1404,** pin **1406,** and pin **1408** have length **1434.** Housing **1402** has length **1436,** height **1438,** and depth **1440.** Length **1436** may be about 5 centimeters, height **1438** may be about 5 centimeters, and depth **1440** may be about 5 centimeters. Of course, housing 1402 may have other dimensions, depending on the particular implementation.

Further, the desired level of accuracy may be a factor in determining the design of soil sensor unit **1400.** In particular, when soil sensor unit **1400** is recoverable, a frequency domain sensor such as a frequency domain capacitive probe may be used in place of a resistive sensor. In this case, the frequency domain capacitive probe may provide a more durable design and more accurate information about soil conditions. However, this type of design may increase the cost of soil sensor unit **1400.**

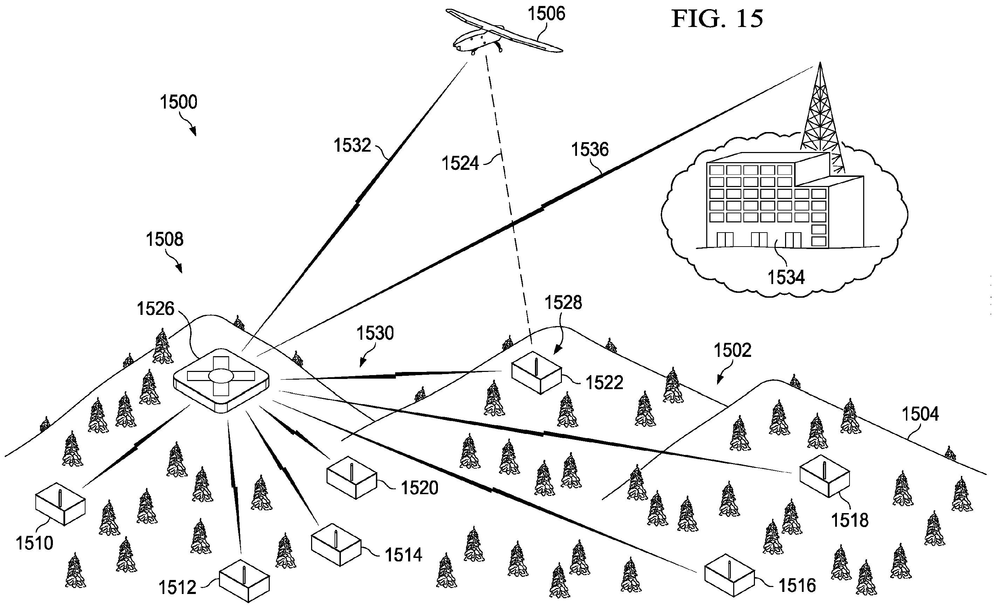

Turning now to **Figure 15****,** an illustration of a forest area is depicted in accordance with an illustrative embodiment. In this depicted example, forest area **1500** is another example of location **1006** in forest **1002** in **Figure 10****.**

As depicted, forest area **1500** is an area in which trees **1502** are present. In this depicted example, ground **1504** in forest area **1500** is hilly or mountainous. In this illustrative example, unmanned aerial vehicle **1506** may deploy soil sensor units **1508** into forest area **1500.** Soil sensor units **1508** include soil sensor unit **1510,** soil sensor unit **1512,** soil sensor unit **1514,** soil sensor unit **1516,** soil sensor unit **1518,** soil sensor unit **1520,** and soil sensor unit **1522.**

These soil sensor units may be deployed various distances apart. These distances may be determined by a desired level of accuracy of information. For example, soil sensor unit **1510** and soil sensor unit **1512** may be one mile apart to achieve a desired level of accuracy of information in these illustrative examples. Of course, soil sensor unit **1510** and soil sensor unit **1512** may be farther apart or closer together, depending on the particular implementation. For example, soil sensor unit **1510** and soil sensor unit **1512** may be one-half mile apart, two miles apart, five miles apart, or some other distance apart in these illustrative examples. As a result, fewer soil sensor units may be used in forest area **1500** to provide a desired level of accuracy of information for soil conditions in ground **1504** than with currently used systems.

With the use of fewer soil sensor units, the cost of generating information about soil in ground **1504** may be reduced. In other illustrative examples, when more soil sensor units **1508** are desired in forest area **1500,** the low cost of soil sensor units **1508** and the higher quality of information generated by soil sensor units **1508** provides more accurate information about soil conditions at a lower cost than with currently used methodologies.

In these illustrative examples, soil sensor units **1508** are configured to generate information about the soil in ground **1504.** This information may be information about a number of conditions of the soil in ground **1504.** In particular, the information may include moisture content.

The moisture content measurements may be used to determine whether soil conditions are favorable for harvesting operations for trees **1502** in forest area **1500.** In particular, in addition to having a desired size, the soil in ground **1504** in forest area **1500** may require a desired moisture level such that equipment moving into forest area **1500** can do so with a desired level of operation. In other words, the moisture content in the soil in ground **1504** may be used to determine whether ground **1504** has a desired stability for equipment that may be used to harvest trees **1502** to operate.

In these illustrative examples, unmanned aerial vehicle **1506** also may deploy transceiver **1526.** Transceiver **1526** may be used to receive information from soil sensor units **1508** and relay or send that information to another location. This location may be at least one of unmanned aerial vehicle **1506,** a manned ground vehicle, control station **1534,** or other suitable locations.

In this illustrative example, unmanned aerial vehicle **1506** may fly over forest area **1500** after deployment of soil sensor units **1508** and obtain information about the soil in ground **1504** from soil sensor units **1508** via transceiver **1526.** The information may include soil information as well as information about soil sensor units **1508.** In particular, the information about soil sensor units **1508** may include the location of soil sensor units **1508.**

As depicted, unmanned aerial vehicle **1506** may fly at a desired distance above ground **1504** to collect information from soil sensor units **1508.** This distance may be determined by the height of the trees, the location of soil sensor units **1508,** the level of power of the transmitter in soil sensor units **1508,** a pre-determined flight pattern for unmanned aerial vehicle **1506,** the type of unmanned aerial vehicles used, or other suitable parameters.

For example, with some types of unmanned aerial vehicles, the unmanned aerial vehicle may fly close to ground **1504** to collect information from soil sensor units **1508.** In these illustrative examples, the height at which unmanned aerial vehicle **1506** may fly over ground **1504** may be determined by the power of the transmitter in soil sensor units **1508** and the sensitivity of the receiver in unmanned aerial vehicle **1506.**

As an example, if soil sensor units **1508** have a transmitter with a range of about two kilometers, unmanned aerial vehicle **1506** may fly at a height less than two kilometers in these illustrative examples. Of course, transmitters with other ranges may be used and thus, unmanned aerial vehicle **1506** may fly at different heights, depending on the particular implementation. At higher altitudes, unmanned aerial vehicle **1506** may fly at higher speeds and may collect information from soil sensor units **1508** more quickly than when flying at lower altitudes.

In these illustrative examples, the location may be identified using global positioning system receivers in soil sensor units **1508.** However, the canopy in trees **1502** may block global positioning system signals from reaching global positioning system receivers in soil sensor units **1508** on ground **1504** in forest area **1500.**

In this instance, the location of soil sensor units **1508** may be identified from trajectories of soil sensor units **1508** as they were deployed from unmanned aerial vehicle **1506.** For example, trajectory **1524** of soil sensor unit **1522** may be used to identify location **1528** of soil sensor unit **1522** on ground **1504.** Trajectory **1524** of soil sensor unit **1522** may be identified from location information transmitted using global positioning system receivers while soil sensor unit **1522** moves along trajectory **1524** above the tree canopy in trees **1502.**

In other illustrative examples, the locations of soil sensor units **1508** may be determined by the global positioning coordinates of unmanned aerial vehicle **1506** at the time of deployment of soil sensor units **1508.** In this case, the location of unmanned aerial vehicle **1506** at the time of deployment of soil sensor units **1508** may provide a desired level of accuracy to receive wireless communications from soil sensor units **1508.** In other words, the range of a transmitter on a soil sensor unit in soil sensor units **1508** may be such that the location of soil sensor units **1508** may be determined with a desired level of accuracy to collect information about soil conditions.

The location and soil information may be sent from soil sensor units **1508** to transceiver **1526** through communications links **1530.** In turn, transceiver **1526** may send this information to another location for analysis. For example, the information may be sent from transceiver **1526** to unmanned aerial vehicle **1506** through wireless communications link **1532.** In another illustrative example, transceiver **1526** may send the information to control station **1534** over wireless communications link **1536.** In these examples, information may be sent to control station **1534** via other sensors in the form of a mesh network. Of course, in some illustrative examples, soil sensor units **1508** may send the information directly to an information collection vehicle when transceiver **1526** is not being used.

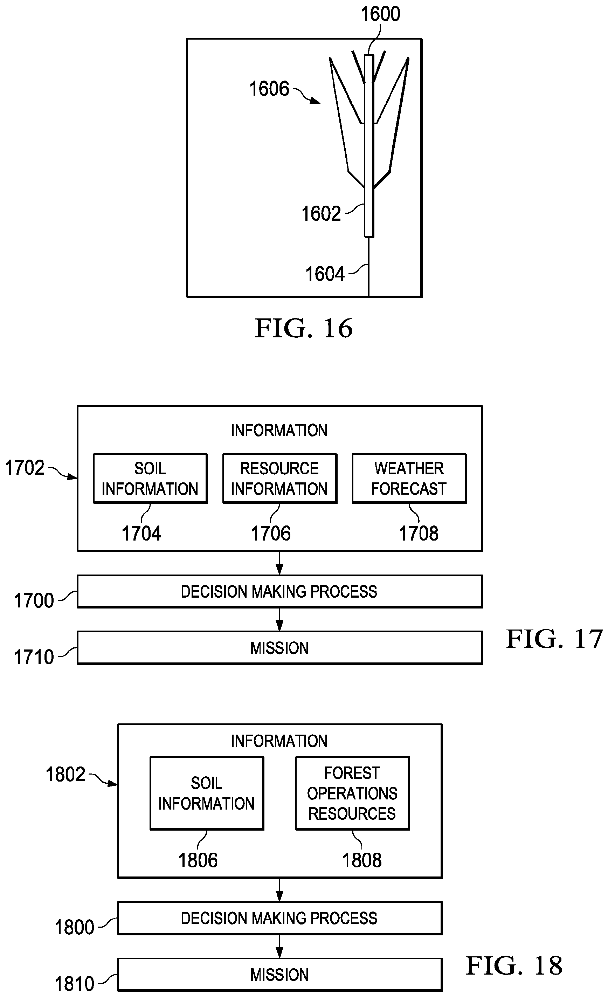

Turning now to **Figure 16****,** an illustration of a soil sensor aerial deployment unit is depicted in accordance with an illustrative embodiment. As depicted, soil sensor unit **1600** is an example of a physical implementation of soil sensor unit **1100** shown in block form in **Figure 11****.** Further, soil sensor unit **1600** may be used to implement one or more of soil sensor units **1508** in **Figure 15****.**

As depicted, soil sensor unit **1600** has a configuration similar to a dart. In this illustrative example, soil sensor unit **1600** has housing **1602.** Probe **1604** is associated with and extends from housing **1602.**

Additionally, soil sensor unit **1600** also includes fins **1606.** The shape of housing **1602,** the configuration of fins **1606,** and the configuration of probe **1604** are configured such that probe **1604** enters the ground when soil sensor unit **1600** is deployed. Further, soil sensor unit **1600** is configured to be deployed in an already established forest, the shape of soil sensor unit **1600** may be such that soil sensor unit **1600** penetrates and passes through a forest canopy.

Soil sensor unit **1600** may include other components within housing **1602.** In this illustrative example, these components may be similar to those shown in other examples of soil sensor units such as soil sensor unit **1300** in **Figure 13** and soil sensor unit **1400** in **Figure 14****.**

The illustration of the deployment of soil sensor units and implementations for soil sensor units in **Figures 13-16** are only meant as examples of some implementations and not mean to limit the manner in which soil sensor units may be deployed or constructed. For example, the soil sensor units may have other shapes such as cubes, pyramids, or other suitable shapes. Additionally, different types of soil sensor units may be used in the same location. In other words, soil sensor units may be heterogeneous in type and not necessarily homogenous.

Turning now to **Figure 17****,** an illustration of a model of decision making for in-filling recently planted areas of a forest is depicted in accordance with an illustrative embodiment. As depicted, decision making process **1700** is an example of a process that may be implemented in forestry manager **1014** in **Figure 10****.**

In this illustrative example, decision making process **1700** may use a number of types of information to perform planting of trees. This information includes information in addition to information **1004** generated by assets **1016** in **Figure 10****.**

As depicted, information **1702** includes soil information **1704,** resource information **1706,** weather forecast **1708,** and other suitable types of information. Foresters may use soil information **1704,** resource information **1706,** weather forecast **1708,** and other suitable types of information to determine a plan for planting trees **1001** in **Figure 10****.** Foresters may use their forestry experiences, as well as information **1702** to make decisions about planting trees **1001.** In other illustrative examples, soil information **1704,** resource information **1706,** weather forecast **1708,** and other suitable types of information may be used by a device with a desired level of intelligence to automate a portion or all of the decision-making process to generate mission **1710.**

In this example, soil information **1704** may include at least one of soil moisture conditions, soil temperature conditions, soil conductivity, nitrogen content, pH, calcium content, salt content, nutrient content, and other suitable types of information about soil conditions. Resource information **1706** may include an identification of at least one of planting equipment, human operators, and other resources that may be used to plant trees. Weather forecast **1708** includes forecasts for the area in which the planting of trees is desired. This weather forecast information may include forecasts for rain, temperature, and other weather conditions.

Mission **1710** is generated by decision making process **1700** using information **1702.** In this illustrative example, mission **1710** is a planting mission and may include at least one of a desired time to plant trees, a type of seedlings, planting density, fertilization strategies, and other suitable types of information. In this manner, decision making process **1700** takes into account than currently used. Currently, obtaining soil information **1704** to make good decisions is cost prohibitive. Thus, the current methods do not support identification of the type of seedlings to use. Further, currently employed decision making processes may not be implemented in hardware such as forestry manager **1014.** Thus, decision making process **1700** takes into account a greater number of different types of factors in generating mission **1710** than currently used decision making processes for planting trees in the forest.

Turning now to **Figure 18****,** an illustration of a model of decision making for harvesting trees is depicted in accordance with an illustrative embodiment. As depicted, decision making process **1800** is an example of a process that may be implemented in forestry manager **1014** in **Figure 10****.** In this illustrative example, decision making process **1800** may use a number of types of information to perform harvesting of trees. This information includes information in addition to information **1004** generated by assets **1016** in **Figure 10****.**

In this example, information **1802** includes soil information **1806.** Soil information **1806** may include a number of soil conditions that indicate the stability of the ground in the area for operating equipment. Additionally, information **1802** also may include forest operations resources **1808.** Forest operation resources **1808** may include an identification of harvesting equipment, trucks for carrying trees, human operators, and other resources that may be used for forest management.

As depicted, decision making process **1800** uses information **1802** to generate mission **1810.** Mission **1810** is a forest operation and may indicate when harvesting may occur. Further, in some illustrative examples, forest operation mission **1810** also may include an identification of what equipment may be used if constraints are present or when operations may occur. For example, if a three month period is provided for harvesting trees, the type of harvesting equipment that may be used may be based on soil conditions identified at different times during the three month period of time. As an example, different equipment may be used during different months or weeks depending on the soil conditions and how the soil conditions affect the stability of the ground with respect to using different types of equipment.

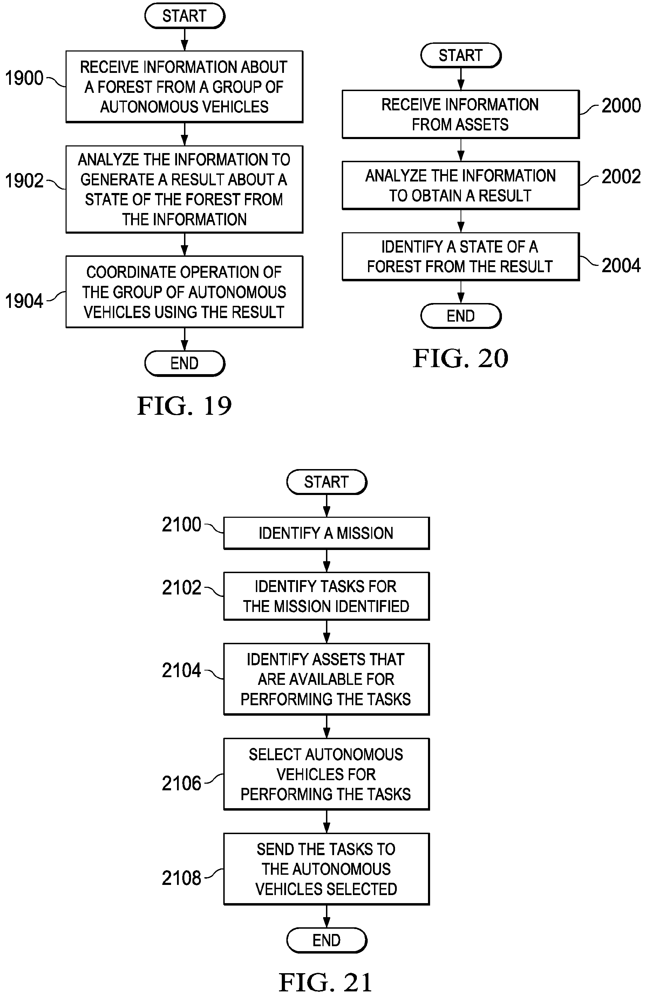

Turning now to **Figure 19****,** an illustration of a flowchart of a process for managing a forest is depicted in accordance with an illustrative embodiment. The process illustrated in **Figure 19** may be implemented in forestry management environment **200** in **Figure 2****.** In particular, the process may be implemented using forestry manager **202** in **Figure 2****.**

The process begins by receiving information about a forest from a group of autonomous vehicles (operation **1900).** The process analyzes the information to generate a result about a state of the forest from the information (operation **1902).** The process then coordinates operation of the group of autonomous vehicles using the result (operation **1904)** with the process terminating thereafter.

Turning now to **Figure 20****,** an illustration of a flowchart of a process for processing information received from assets is depicted in accordance with an illustrative embodiment. The process illustrated in **Figure 20** may be implemented in forestry manager **202** in **Figure 2****.**

The process begins by receiving information from assets (operation **2000).** In these illustrative examples, the assets may take various forms. In particular, the assets may be a group of autonomous vehicles that may operate to collect information without human intervention. Specifically, the group of autonomous vehicles may operate as a swarm or as a group of swarms.

The information is analyzed to obtain a result (operation **2002).** A state of a forest is identified from the result (operation **2004)** with the process terminating thereafter. In these illustrative examples, the result may take various forms such as identifying a state of forest health, forest inventory, safety risks, illegal activity, and other states.

With reference now to **Figure 21****,** an illustration of a flowchart of a process for coordinating the operation of assets is depicted in accordance with an illustrative embodiment. The process illustrated in **Figure 21** may be implemented in forestry manager **202** in **Figure 2****.** Further, this process may be implemented to use assets **204** such as group of autonomous vehicles **226** in **Figure 2****.**

The process begins by identifying a mission (operation **2100).** This mission may be identified based on at least one of user input, a state of the forest, and other suitable information. For example, user input may select a particular mission to be performed in the forest. In other examples, forestry manager **202** may generate missions based on the state of the forest.

The process identifies tasks for the mission identified (operation **2102).** These tasks may be obtained from a preselected template of tasks for missions. In other illustrative examples, the tasks may be generated by forestry manager **202** when forestry manager **202** has a level of intelligence that allows for formulating tasks. For example, forestry manager **202** may implement artificial intelligence processes. Next, the process identifies assets that are available for performing the tasks (operation **2104).** In these illustrative examples, the assets may be a portion or all of a group of autonomous vehicles that are available for use by the forestry manager.

The process then selects autonomous vehicles for performing the tasks (operation **2106).** In these illustrative examples, each autonomous vehicle may be assigned a task or a group of autonomous vehicles may be assigned one or more tasks to perform the tasks as a swarm. The process then sends the tasks to the autonomous vehicles selected (operation **2108)** with the process terminating thereafter.

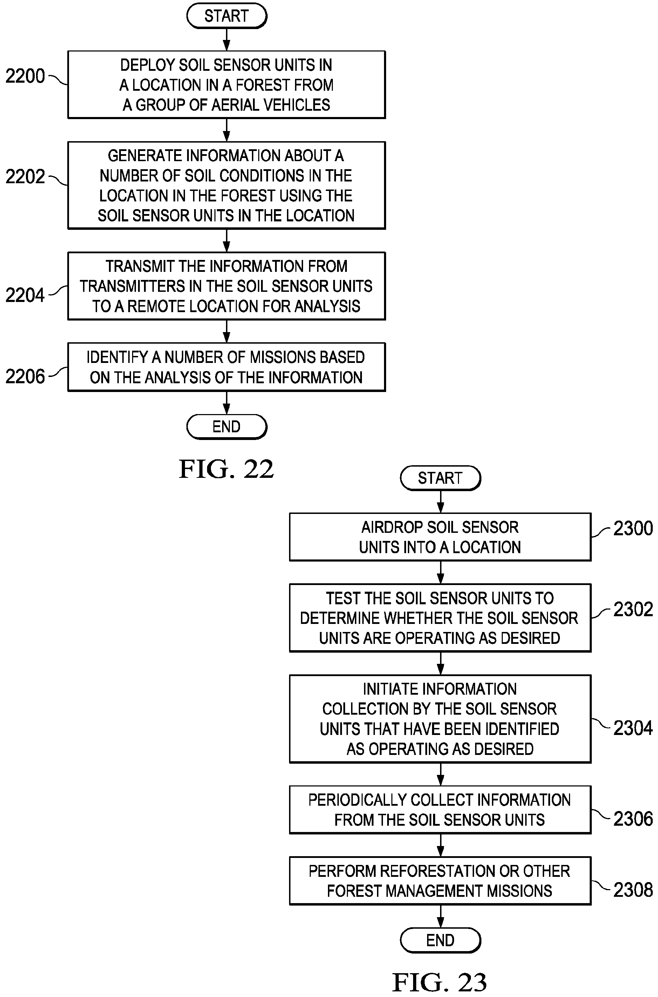

Turning now to **Figure 22****,** an illustration of a flowchart of a process for managing a location is depicted in accordance with an illustrative embodiment. The process illustrated in **Figure 22** may be used to manage a location such as a forest area. In this example, the forest area may be location **1006** in forest **1002** in forest management environment **1000** in **Figure 10****.** Further, the process illustrated in **Figure 22** may be implemented using forestry management system **1003** in **Figure 10****.**

The process begins by deploying soil sensor units in a location in a forest from a group of aerial vehicles (operation **2200).** In this illustrative example, a group of items means one or more items. For example, a group of aerial vehicles is one or more aerial vehicles. In this case, an aerial vehicle in the group of aerial vehicles may be selected from one of an unmanned aerial vehicle and a manned aerial vehicle. Both manned and unmanned aerial vehicles may be included in the group of aerial vehicles, depending on the particular implementation.

The process then generates information about a number of soil conditions in the location in the forest using the soil sensor units in the location (operation **2202).** The process then transmits the information from transmitters in the soil sensor units to a remote location for analysis (operation **2204).** Based on the analysis of the information, a number of missions may be identified (operation **2206)** with the process terminating thereafter. The identification of the mission may merely identify the type of mission needed. In other illustrative examples, the identification of the mission may include identifying tasks and assets for performing the mission. In these illustrative examples, the number of missions may include at least one of a planting mission, a harvesting mission, a soil condition identification mission, a fire condition warning mission, a forest maintenance mission, and a forest inventory mission.

Turning now to **Figure 23****,** an illustration of a flowchart of a process for obtaining information about a number of soil conditions in a location in a forest is depicted in accordance with an illustrative embodiment. The process illustrated in **Figure 23** is another example of a process that may be used to obtain information **1004** about location **1006** in forest **1002** in **Figure 10****.**

The process begins by airdropping soil sensor units into a location (operation **2300).** The process then tests the soil sensor units to determine whether the soil sensor units are operating as desired (operation **2302).** This testing may be used to determine whether the soil sensor units are operating and are in desired locations. For example, if a soil sensor unit does not penetrate the ground, the information generated may not be as accurate as desired. This identification may be made by determining whether other soil sensor units around or in a location near the soil sensor unit are generating information about soil conditions that are within an expected range for operating correctly.

In another illustrative example, the testing may determine whether the soil sensor unit has landed properly and penetrated the ground as opposed to bouncing off an object such as a log, tree, rock, or merely landed on the ground without penetrating. For example, the testing of the soil sensor units may include obtaining information from an orientation sensor to determine the orientation of the soil sensor unit. A vertical orientation may imply that the soil sensor unit has penetrated the ground. A non-vertical orientation may imply that the soil sensor unit may have landed on the ground without penetrating the ground.

As another example, information may be obtained from a photometer in the soil sensor unit to determine whether the soil sensor unit has penetrated the ground. If no light is detected, an implication may be made that the soil sensor unit has penetrated the ground. If the photometer indicates that some light is detected, then the soil sensor unit may have penetrated the canopy of the forest but not the ground.

The process then initiates information collection by the soil sensor units that have been identified as operating as desired (operation **2304).** This initiation may be generated by signals sent from a source such as an unmanned aerial vehicle, a transceiver, or some other suitable device.

The process then periodically collects information from the soil sensor units (operation **2306).** This periodical collection may occur in a number of different ways. For example, the soil sensor units may be configured to transmit information periodically during selected time intervals. In other illustrative examples, the information may be collected by sending signals to the soil sensor units collecting the information.

Thereafter, reforestation or other forest management missions may be performed (operation **2308)** with the process terminating thereafter. The harvesting operations include harvesting trees. Additionally, recovery operations may be performed to recover soil sensor units and any transceivers that are in the area. The soil sensor units and transceivers may then be used in other airdrops for other locations to determine whether conditions are present for harvesting trees in those locations.

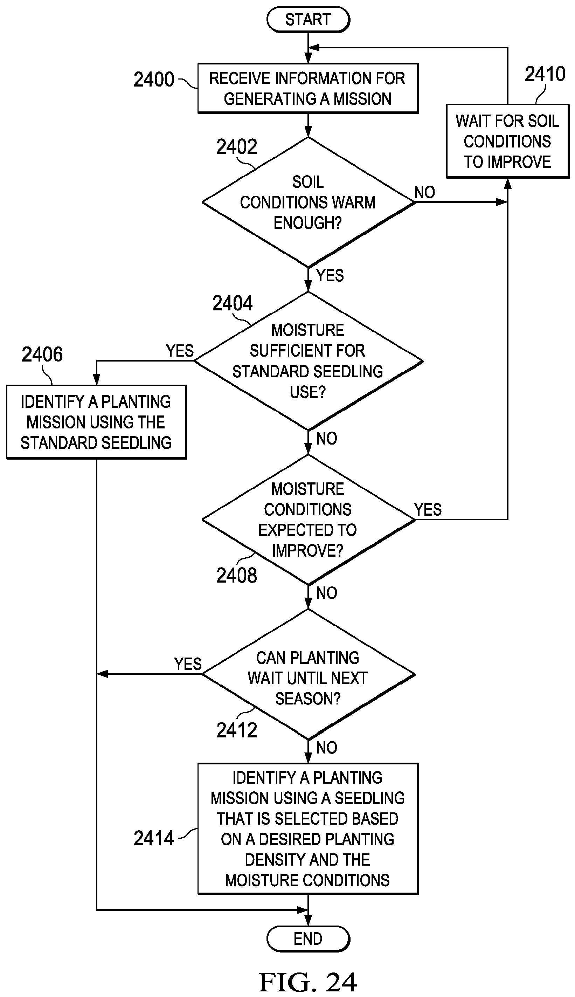

Turning now to **Figure 24****,** an illustration of a flowchart of a decision making process for generating a mission is depicted in accordance with an illustrative embodiment. The process illustrated in **Figure 24** is an example of operations that may be performed by decision making process **1700** in **Figure 17** when implemented in forestry manager **1014** in **Figure 10****.**

The process begins by receiving information for generating a mission (operation **2400).** This information may include, for example, without limitation, information about a number of soil conditions, harvesting resources, weather forecasts, and other suitable information.

A determination is made as to whether the soil conditions are warm enough for planting seedlings (operation **2402).** The determination in operation **2402** may be made using soil information received from soil sensor units.

If the soil conditions are warm enough, a determination is made as to whether the moisture in the soil is sufficient for using a standard seedling (operation **2404).** The information about the moisture in the soil also may be in soil information received from soil sensor units. If the soil condition is sufficient for using the standard seedling, a planting mission is identified using the standard seedling (operation **2406)** with the process terminating thereafter.

Otherwise, a determination is made as to whether moisture conditions are expected to improve within a selected period of time (operation **2408).** The determination in **2408** may be made using weather forecast information. The selected period of time may be one selected based on available resources, harvesting requirements, and other factors. The selected period of time may be a week, a month, or some other suitable period of time.

If the soil conditions are expected to improve, the process waits for the soil conditions to improve (operation **2410)** with the process returning to operation **2400** as described above. This mission may be used to obtain more soil information at a later period of time.

If the moisture conditions are not expected to improve within the selected period of time, a determination is made as to whether the planting of trees can wait until the next season (operation **2412).** If the planting of seedlings can wait until the next season, the process terminates. Otherwise, a planting mission is identified using a seedling that is selected based on a desired planting density and the moisture conditions (operation **2414)** with the process terminating thereafter. The selected planting density may be a planting density to account for greater expected mortality. The seedling selected for planting in operation **2414** may be a containerized seedling or may be some other type of seedling, depending on the particular implementation.

With reference again to operation **2402,** if the soil conditions are not warm enough, the process returns to operation **2410** to identify a forest inventory mission.

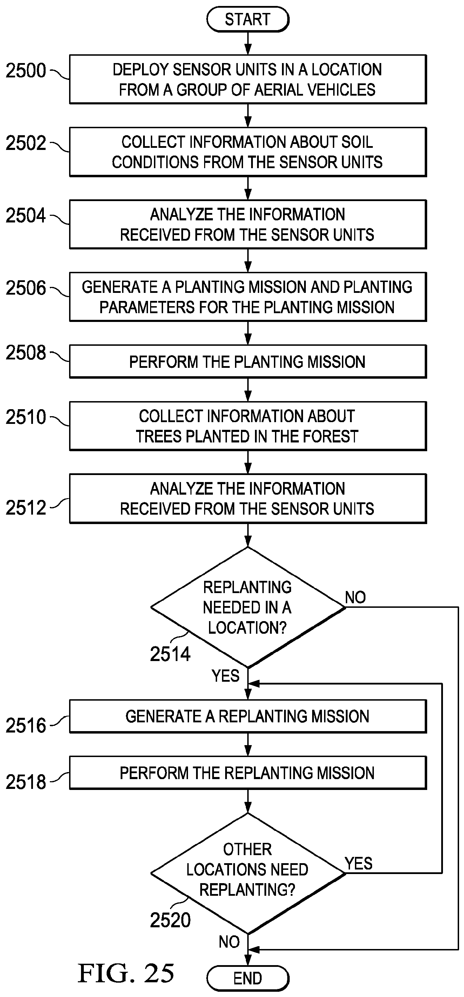

With reference now to **Figure 25****,** an illustration of a flowchart of a decision making process for generating and performing a mission is depicted in accordance with an illustrative embodiment. The process illustrated in **Figure 25** is an example of operations that may be performed by decision making process **1700** in **Figure 17** when implemented in forestry manager **1014** in **Figure 10****.** Further, the process illustrated in this figure may be used by assets **1016** to perform missions **1030** in **Figure 10****.**

The process begins by deploying sensor units in a location from a group of aerial vehicles (operation **2500).** The location may be location **1006** in forest **1002** in **Figure 10****.** The location may be in unplanted forest land in this illustrative example. The sensor units deployed by the group of aerial vehicles may be soil sensor units or other types of sensor units, depending on the particular implementation.

Next, the process collects information about soil conditions from the sensor units (operation **2502).** The information received from the sensor units is analyzed (operation **2504).** The process then generates a planting mission and planting parameters for the planting mission (operation **2506).** These planting parameters may be planting time, type of seedlings used, spacing of trees, quantity of fertilizer needed, type of fertilizer needed, or other suitable types of parameters for planting of trees in an unplanted forest area.

The planting mission is performed (operation **2508).** After a period of time, information about trees planted in the forest is collected by the sensor units (operation **2510).** This information may include planting density of seedlings planted in the forest, rate of growth of the seedlings, soil conditions, or other types of information. The information received from the sensor units is analyzed (operation **2512).**

Next, a determination is made as to whether replanting is needed in a location in the forest (operation **2514).** Replanting may be needed in sparse areas of the forest, in areas where seedlings are not growing as desired, in areas where the forest is not as dense as desired, or a combination thereof.

If replanting is needed, the process generates a replanting mission based on the analysis of the information received from the sensor units (operation **2516).** This replanting mission may include parameters such as time of replanting, type of seedlings used, location of replanting, quantity of fertilizer needed, and other parameters. The replanting mission is performed (operation **2518).** Next, a determination is made as to whether other locations in the forest need replanting (operation **2520).**

If other locations of the forest need replanting, the process returns to operation **2516** as described above. Otherwise, the process terminates. With reference again to operation **2514,** if replanting is not needed, the process terminates.

Thus, the deployment of sensor units from the group of unmanned aerial vehicles with an illustrative embodiment provides information about the forest to generate a number of different types of missions. These missions may be performed to more easily and successfully plant trees, replant trees, conduct a forest inventory, or harvest trees in a location in the forest. Further, cost will be reduced because weather conditions, soil conditions, and other factors may be taken into account in generating the appropriate missions for planting and replanting trees in the forest.

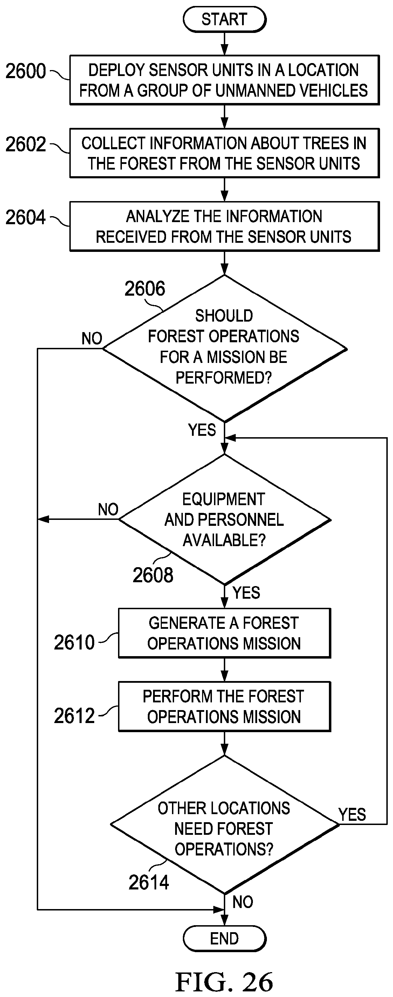

With reference now to **Figure 26****,** an illustration of a flowchart of a decision making process for generating and performing forest operations in a mission is depicted in accordance with an illustrative embodiment. The process illustrated in **Figure 26** is an example of operations that may be performed by decision making process **1800** in **Figure 18** when implemented in forestry manager **1014** in **Figure 10****.** Further, the process illustrated in this figure may be used by assets **1016** to perform missions **1030** in **Figure 10****.**

The process begins by deploying sensor units in a location from a group of unmanned vehicles (operation **2600).** The location may be location **1006** in forest **1002** in **Figure 10****.** The location may be a location in the forest in which forest operations may be performed. The sensor units deployed by the group of unmanned vehicles may be soil sensor units or other types of sensor units, depending on the particular implementation. The group of unmanned vehicles may be low-flying unmanned aerial vehicles. In other illustrative examples, the group of unmanned vehicles may include unmanned ground vehicles and other suitable types of vehicles.

Next, the process collects information about trees in the forest from the sensor units (operation **2602).** The information received from the sensor units is analyzed (operation **2604).**

The process then determines if forest operations for a mission should be performed in a location in the forest (operations **2606).** The forest operations may be one of inspection, core sampling, measuring, thinning, harvesting, and other suitable types of forest operations. If a forest operation should be performed, the process then determines if equipment and personnel are available to perform the forest operation (operation **2608).** If equipment and personnel are available, the process generates a forest operations mission (operation **2610).** The forest operations mission may include tasks for each of assets **1016** to perform. For example, with harvesting, the forest operations mission may specify tasks to be performed by harvesting equipment and forestry personnel.

Next, the forest operations mission is performed (operation **2612).** A determination is then made as to whether other locations in the forest need forest operations to be performed (operation **2614).**

If other locations of the forest need forest operations to be performed, the process returns to operation **2608** as described above. Otherwise, the process terminates.

With reference again to operation **2606,** if forest operations should not be performed, the process terminates. Returning to operation **2608,** if equipment and personnel are not available to perform the forest operation, the process also terminates.

Thus, the illustrative embodiments provide a means of measuring soil conditions over vast areas at low cost by utilizing remote sensors and autonomous systems. In particular, the system may improve productivity of reforestation activities by reducing costs and improving seedling yield through greater optimization of reforestation parameters. This optimization may be enabled by more accurate soil moisture and temperature data prior to and at the time of planting. The automated system makes this data available to computer programs and analysts in a timely and cost effective manner.

With the use of an illustrative embodiment, soil conditions are available in real time to aid in decision making. Information may be used to determine timing of planting, seedling type selection, and selection of planting density for reforestation operations. The sensor units deployed by the unmanned aerial vehicles and other types of unmanned vehicles may monitor conditions after the initial planting of trees in the forest to determine locations in the forest that should be in-filled due to poor initial growth conditions or high seedling mortality rates.

Although the illustrative embodiments have been described with regard to artificial regeneration by planting and replanting trees in a forest, the illustrative embodiments may also apply to natural regeneration of trees in the forest. For example, the illustrative embodiments may be used to monitor conditions and provide information about root suckering, stump sprouting, natural seedlings, or other suitable indications of natural regeneration of the forest. Further, when an illustrative embodiment is used for planting or replanting trees in a forest, the illustrative embodiments may aid in planting using seedlings, machine planting, hand planting, or some other suitable type of artificial regeneration of the forest.

Additionally, although the illustrative embodiments have been described as being used for forestry management operations, the forestry management system may also be applied to the management of a number of other domains. These domains may include precision agriculture, hydrological research, and monitoring soil salt levels due to human activity, large scale construction such as open pit mining, and other suitable activities.

The flowcharts and block diagrams in the different depicted embodiments illustrate the architecture, functionality, and operation of some possible implementations of apparatuses and methods in an illustrative embodiment. In this regard, each block in the flowcharts or block diagrams may represent a module, a segment, a function, and/or a portion of an operation or step. For example, one or more of the blocks may be implemented as program code, in hardware, or a combination of the program code and hardware. When implemented in hardware, the hardware may, for example, take the form of integrated circuits that are manufactured or configured to perform one or more operations in the flowcharts or block diagrams.

In some alternative implementations of an illustrative embodiment, the function or functions noted in the blocks may occur out of the order noted in the figures. For example, in some cases, two blocks shown in succession may be executed substantially concurrently, or the blocks may sometimes be performed in the reverse order, depending upon the functionality involved. Also, other blocks may be added in addition to the illustrated blocks in a flowchart or block diagram.

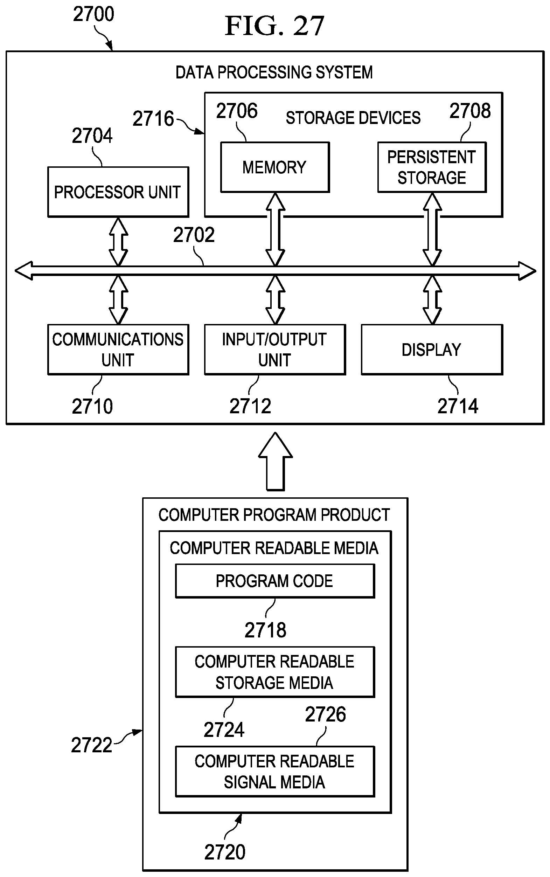

Turning now to **Figure 27****,** an illustration of a block diagram of a data processing system is depicted in accordance with an illustrative embodiment. Data processing system **2700** may be used to implement computer system **210** in **Figure 2****,** controller **610** in **Figure 6****,** identifier **810** in **Figure 8****,** and other suitable devices within forestry management environment **200.**

In this illustrative example, data processing system **2700** includes communications framework **2702,** which provides communications between processor unit **2704,** memory **2706,** persistent storage **2708,** communications unit **2710,** input/output unit **2712,** and display **2714.** In this example, communication framework may take the form of a bus system.

Processor unit **2704** serves to execute instructions for software that may be loaded into memory **2706.** Processor unit **2704** may be a number of processors, a multi-processor core, or some other type of processor, depending on the particular implementation.

Memory **2706** and persistent storage **2708** are examples of storage devices **2716.** A storage device is any piece of hardware that is capable of storing information, such as, for example, without limitation, data, program code in functional form, and/or other suitable information either on a temporary basis and/or a permanent basis. Storage devices **2716** may also be referred to as computer readable storage devices in these illustrative examples. Memory **2706,** in these examples, may be, for example, a random access memory or any other suitable volatile or non-volatile storage device. Persistent storage **2708** may take various forms, depending on the particular implementation.

For example, persistent storage **2708** may contain one or more components or devices. For example, persistent storage **2708** may be a hard drive, a flash memory, a rewritable optical disk, a rewritable magnetic tape, or some combination of the above. The media used by persistent storage **2708** also may be removable. For example, a removable hard drive may be used for persistent storage **2708.**

Communications unit **2710,** in these illustrative examples, provides for communications with other data processing systems or devices. In these illustrative examples, communications unit **2710** is a network interface card.

Input/output unit **2712** allows for input and output of data with other devices that may be connected to data processing system **2700.** For example, input/output unit **2712** may provide a connection for user input through a keyboard, a mouse, and/or some other suitable input device. Further, input/output unit **2712** may send output to a printer. Display **2714** provides a mechanism to display information to a user.

Instructions for the operating system, applications, and/or programs may be located in storage devices **2716,** which are in communication with processor unit **2704** through communications framework **2702.** The processes of the different embodiments may be performed by processor unit **2704** using computer-implemented instructions, which may be located in a memory, such as memory **2706.**

These instructions are referred to as program code, computer usable program code, or computer readable program code that may be read and executed by a processor in processor unit **2704.** The program code in the different embodiments may be embodied on different physical or computer readable storage media, such as memory **2706** or persistent storage **2708.**

Program code **2718** is located in a functional form on computer readable media **2720** that is selectively removable and may be loaded onto or transferred to data processing system **2700** for execution by processor unit **2704.** Program code **2718** and computer readable media **2720** form computer program product **2722** in these illustrative examples. In one example, computer readable media **2720** may be computer readable storage media **2724** or computer readable signal media **2726.**

In these illustrative examples, computer readable storage media **2724** is a physical or tangible storage device used to store program code **2718** rather than a medium that propagates or transmits program code **2718.**

Alternatively, program code **2718** may be transferred to data processing system **2700** using computer readable signal media **2726.** Computer readable signal media **2726** may be, for example, a propagated data signal containing program code **2718.** For example, computer readable signal media **2726** may be an electromagnetic signal, an optical signal, and/or any other suitable type of signal. These signals may be transmitted over communications links, such as wireless communications links, optical fiber cable, coaxial cable, a wire, and/or any other suitable type of communications link.

The different components illustrated for data processing system **2700** are not meant to provide architectural limitations to the manner in which different embodiments may be implemented. The different illustrative embodiments may be implemented in a data processing system including components in addition to and/or in place of those illustrated for data processing system **2700.** Other components shown in **Figure 27** can be varied from the illustrative examples shown. The different embodiments may be implemented using any hardware device or system capable of running program code **2718.**

Thus, the illustrative embodiments provide a method and apparatus for managing a forest. In the illustrative examples, a forestry management system may gather information about a forest from autonomous vehicles and analyze that information more efficiently than currently used systems in which human operators collect information about a forest.

Further, the illustrative embodiments also generate missions based on a current state of the forest as well as from user input. These missions may be sent to one or more autonomous vehicles. These missions may include information gathering or state changes to be implemented in the forest. Information gathering may be performed for various purposes in managing the forest. These purposes include maintaining a health of the forest, identifying inventory in the forest, identifying safety risks in the forest, identifying illegal activities in the forest, and other purposes. The effect of changing states in the forest may include fighting fires, pest control, harvesting, and other suitable state changes.

With the use of autonomous vehicles and the capability of having autonomous vehicles cooperate with each other in performing tasks in a swarm, the illustrative examples provide more efficient mechanisms for collecting information, affecting changes, or a combination thereof with respect to a forest.

Further, the use of autonomous vehicles and sensor systems in the illustrative embodiments may allow for a desired level of sampling of information from a sufficient number of locations to obtain results that are more accurate than currently possible. The illustrative embodiments also allow for action to be taken in response to the results that may be more timely and accurate than currently possible.

Further, the illustrative embodiments may avoid issues that result from interpretations of observations made by personnel to generate information about the forest. The use of at least one of unmanned vehicles and sensor systems in the illustrative embodiments results in information being generated in a manner that is less subjective as compared to how information is generated by personnel in the forest.

The description of the different illustrative embodiments has been presented for purposes of illustration and description, and is not intended to be exhaustive or limited to the embodiments in the form disclosed. Many modifications and variations will be apparent to those of ordinary skill in the art.

Further, different illustrative embodiments may provide different features as compared to other illustrative embodiments. The embodiment or embodiments selected are chosen and described in order to best explain the principles of the embodiments, the practical application, and to enable others of ordinary skill in the art to understand the disclosure for various embodiments with various modifications as are suited to the particular use contemplated.

## Claims

1. A forestry management system comprising:
sensor units configured to be deployed in a location, generate information about a number of soil conditions in the location, and transmit the information using wireless communications links; and
a group of aerial vehicles configured to carry the sensor units and deploy the sensor units in the location,
wherein the sensor units comprise soil sensor units (1028), the soil sensor units being bottom heavy comprising pins (1214) and housing (1102), the pins and housing being weighted such that the pins extend into the ground when the soil sensor units are deployed through an airdrop, the sensor units implemented using the pins.

2. The forestry management system of claim 1 further comprising:
a base station configured to receive the information from the sensor units in the location over the wireless communications links and send the information to a remote location over another wireless communications link.

3. The forestry management system of claim 3, wherein the base station is powered by a solar power generation system.

4. The forestry management system of any of claims 1 to 3 further comprising:
a forestry manager configured to receive the information relating to the number of soil conditions for the location in a forest from the sensor units deployed by the group of aerial vehicles and identify a mission based on the number of soil conditions.

5. The forestry management system of any of claims 1 to 4, wherein an aerial vehicle in the group of aerial vehicles is selected from one of an unmanned aerial vehicle and a manned aerial vehicle.

6. A method for managing a forestry location comprising:
a group of aerial vehicles carrying and deploying a plurality of sensor units in the location;
wherein when deployed, the sensor units:
generate information about a number of soil conditions in the location; and
transmit the information using wireless communications links;
wherein the sensor units comprise soil sensor units (1028), the soil sensor units being bottom heavy comprising pins (1214) and housing (1102), the pins and housing being weighted such that the pins extend into the ground when the soil sensor units are deployed through an airdrop, the sensor units implemented using the pins.

7. The method of claim 6, further comprising:
a base station receiving the information from the sensor units in the location over the wireless communications links; and
sending the information to a remote location over another wireless communications link.

8. The method of claim 7, wherein the base station is powered by a solar power generation system.

9. The method of any of claims 6 to 8 further comprising:
a forestry manager receiving the information relating to the number of soil conditions for the location in a forest from the sensor units deployed by the group of aerial vehicles; and
identifying a mission based on the number of soil conditions.

10. The method of any of claims 6 to 9, wherein an aerial vehicle in the group of aerial vehicles is selected from one of an unmanned aerial vehicle and a manned aerial vehicle.

## Patentansprüche

1. Forstbewirtschaftungssystem, umfassend:
Sensoreinheiten, die ausgelegt sind, an einem Ort eingesetzt zu werden, Informationen über eine Anzahl von Bodenbedingungen an dem Ort zu erzeugen und die Informationen unter Verwendung drahtloser Kommunikationsverbindungen zu übertragen; und
eine Gruppe von Luftfahrzeugen, die ausgelegt sind, die Sensoreinheiten zu tragen und die Sensoreinheiten an dem Ort einzusetzen,
wobei die Sensoreinheiten Bodensensoreinheiten (1028) umfassen, wobei die Bodensensoreinheiten bodenlastig sind und Stifte (1214) sowie ein Gehäuse (1102) umfassen, wobei die Stifte und das Gehäuse derart gewichtet sind, dass sich die Stifte in den Boden erstrecken, wenn die Bodensensoreinheiten durch einen Luftabwurf eingesetzt werden, wobei die Sensoreinheiten unter Verwendung der Stifte implementiert sind.

2. Forstbewirtschaftungssystem nach Anspruch 1, ferner umfassend:
eine Basisstation, die ausgelegt ist, die Informationen von den Sensoreinheiten an dem Ort über die drahtlosen Kommunikationsverbindungen zu empfangen und die Informationen über eine weitere drahtlose Kommunikationsverbindung an einen entfernten Ort zu senden.

3. Forstbewirtschaftungssystem nach Anspruch 3, wobei die Basisstation durch ein solares Energieerzeugungssystem mit Energie versorgt wird.

4. Forstbewirtschaftungssystem nach einem der Ansprüche 1 bis 3, ferner umfassend:
einen Forstbewirtschafter, der ausgelegt ist, die Informationen bezüglich der Anzahl von Bodenbedingungen für den Ort in einem Wald von den durch die Gruppe von Luftfahrzeugen eingesetzten Sensoreinheiten zu empfangen und einen Einsatz basierend auf der Anzahl von Bodenbedingungen zu identifizieren.

5. Forstbewirtschaftungssystem nach einem der Ansprüche 1 bis 4, wobei ein Luftfahrzeug in der Gruppe von Luftfahrzeugen aus einem unbemannten Luftfahrzeug und einem bemannten Luftfahrzeug ausgewählt ist.

6. Verfahren zum Bewirtschaften eines Forstorts, umfassend:
eine Gruppe von Luftfahrzeugen, die eine Mehrzahl von Sensoreinheiten an dem Ort trägt und einsetzt;
wobei die Sensoreinheiten, wenn sie eingesetzt sind:
Informationen über eine Anzahl von Bodenbedingungen an dem Ort erzeugen; und
die Informationen unter Verwendung drahtloser Kommunikationsverbindungen übertragen;
wobei die Sensoreinheiten Bodensensoreinheiten (1028) umfassen, wobei die Bodensensoreinheiten bodenlastig sind und Stifte (1214) sowie ein Gehäuse (1102) umfassen, wobei die Stifte und das Gehäuse derart gewichtet sind, dass sich die Stifte in den Boden erstrecken, wenn die Bodensensoreinheiten durch einen Luftabwurf eingesetzt werden, wobei die Sensoreinheiten unter Verwendung der Stifte implementiert sind.

7. Verfahren nach Anspruch 6, ferner umfassend:
Empfangen der Informationen von den Sensoreinheiten an dem Ort über die drahtlosen Kommunikationsverbindungen durch eine Basisstation; und
Senden der Informationen an einen entfernten Ort über eine weitere drahtlose Kommunikationsverbindung.

8. Verfahren nach Anspruch 7, wobei die Basisstation durch ein solares Energieerzeugungssystem mit Energie versorgt wird.

9. Verfahren nach einem der Ansprüche 6 bis 8, ferner umfassend:
Empfangen der Informationen bezüglich der Anzahl von Bodenbedingungen für den Ort in einem Wald von den durch die Gruppe von Luftfahrzeugen eingesetzten Sensoreinheiten durch einen Forstbewirtschafter; und
Identifizieren eines Einsatzes basierend auf der Anzahl von Bodenbedingungen.

10. Verfahren nach einem der Ansprüche 6 bis 9, wobei ein Luftfahrzeug in der Gruppe von Luftfahrzeugen aus einem unbemannten Luftfahrzeug und einem bemannten Luftfahrzeug ausgewählt ist.

## Revendications

1. Système de gestion de foresterie, comprenant :
des unités capteurs configurées pour être déployées dans un endroit, générer des informations concernant un nombre de conditions de sol dans l'endroit, et transmettre les informations en utilisant des liaisons de communications sans fil ; et
un groupe de véhicules aériens configurés pour transporter les unités capteurs et déployer les unités capteurs dans l'endroit,
dans lequel les unités capteurs comprennent des unités capteurs de sol (1028), les unités capteurs de sol étant lourdes au fond, comprenant des pieux (1214) et un boîtier (1102), les pieux et le boîtier étant lestés de manière telle que les pieux s'étendent dans la terre lorsque les unités capteurs de sol sont déployées par le biais d'un largage aérien, les unités capteurs étant mises en œuvre en utilisant les pieux.

2. Système de gestion de foresterie de la revendication 1, comprenant en outre :
une station de base configurée pour recevoir les informations en provenance des unités capteurs dans l'endroit par le biais des liaisons de communications sans fil et envoyer les informations à un endroit à distance par le biais d'une autre liaison de communications sans fil.

3. Système de gestion de foresterie de la revendication 3, dans lequel la station de base est alimentée par un système de production d'énergie électrique solaire.

4. Système de gestion de foresterie de quelconques des revendications 1 à 3, comprenant en outre :
un gestionnaire de foresterie configuré pour recevoir les informations concernant le nombre de conditions de sol pour l'endroit dans une forêt en provenance des unités capteurs déployées par le groupe de véhicules aériens et identifier une mission sur la base du nombre de conditions de sol.

5. Système de gestion de foresterie de quelconques des revendications 1 à 4, dans lequel un véhicule aérien dans le groupe de véhicules aériens est sélectionné parmi un d'un véhicule aérien sans pilote et d'un véhicule aérien avec pilote.

6. Procédé pour gérer un endroit de foresterie, comprenant :
un groupe de véhicules aériens transportant et déployant une pluralité d'unités capteurs dans l'endroit ;
dans lequel lorsqu'elles sont déployées, les unités capteurs :
génèrent des informations concernant un nombre de conditions de sol dans l'endroit ; et
transmettent les informations en utilisant des liaisons de communications sans fil ;
dans lequel les unités capteurs comprennent des unités capteurs de sol (1028), les unités capteurs de sol étant lourdes au fond, comprenant des pieux (1214) et un boîtier (1102), les pieux et le boîtier étant lestés de manière telle que les pieux s'étendent dans la terre lorsque les unités capteurs de sol sont déployées par le biais d'un largage aérien, les unités capteurs étant mises en œuvre en utilisant les pieux.

7. Procédé de la revendication 6, comprenant en outre :
une station de base recevant les informations en provenance des unités capteurs dans l'endroit par le biais des liaisons de communications sans fil ; et
envoyant les informations à un endroit à distance par le biais d'une autre liaison de communications sans fil.

8. Procédé de la revendication 7, dans lequel la station de base est alimentée par un système de production d'énergie électrique solaire.

9. Procédé de quelconques des revendications 6 à 8, comprenant en outre :
un gestionnaire de foresterie recevant les informations concernant le nombre de conditions de sol pour l'endroit dans une forêt en provenance des unités capteurs déployées par le groupe de véhicules aériens ; et
identifiant une mission sur la base du nombre de conditions de sol.

10. Procédé de quelconques des revendications 6 à 9, dans lequel un véhicule aérien dans le groupe de véhicules aériens est sélectionné parmi un d'un véhicule aérien sans pilote et d'un véhicule aérien avec pilote.
